# EUROPEAN PATENT APPLICATION

(11) **EP 4 079 851 A1**
(43) Date of publication of application: **26.10.2022**
(21) Application number: 20900988.5
(22) Date of filing: 07.12.2020
(51) Int. Cl.: C12N 15/09, C12Q 1/686, C12Q 1/689, G01N 33/53, G01N 33/536, G01N 33/569

(54) **PRIMER SET FOR DETECTING MYCOBACTERIUM TUBERCULOSIS, MYCOBACTERIUM AVIUM, AND MYCOBACTERIUM INTRACELLULARE AND METHOD USING SAME, AND REAGENT KIT THEREFOR**

(30) Priority: 18.12.2019 JP 2019228461
(71) Applicant: FUJIFILM Corporation, Tokyo 106-8620 (JP)
(72) Inventor: NAKAMURA, Tatsuya, Amagasaki-shi Hyogo 661-0963 (JP); TERASHIMA, Kazuhiro, Amagasaki-shi Hyogo 661-0963 (JP)
(74) Representative: Klunker IP Patentanwälte PartG mbB
(86) International application number: PCT/JP2020/045523
(87) International publication number: WO 2021/124960

(57) **Abstract**

An object of the present invention is to provide a method that enables detection of the genus *Mycobacterium* present in a sample even in a case where a difference in concentration (number of copies) is found between the species of the genus *Mycobacterium.*

Provided is "a primer set comprising (i) a primer pair for detecting *Mycobacterium tuberculosis* which consists of a combination of a forward primer consisting of a base sequence represented by SEQ ID NO: 1 and a reverse primer consisting of a base sequence represented by SEQ ID NO: 2, (ii) a primer pair for detecting *Mycobacterium avium* which consists of a combination of a forward primer consisting of a base sequence represented by SEQ ID NO: 3 and a reverse primer consisting of a base sequence represented by SEQ ID NO: 4, and (iii) a primer pair for detecting *Mycobacterium intracellulare* which consists of a combination of a forward primer consisting of a base sequence represented by SEQ ID NO: 5 and a reverse primer consisting of a base sequence represented by SEQ ID NO: 6."

## Description

### Technical Field

The present invention relates to a primer set for detecting *Mycobacterium tuberculosis, Mycobacterium avium,* and *Mycobacterium intracellulare* and a method using the same, and a reagent kit therefor.

### Background Art

Mycobacteriosis such as tuberculosis or nontuberculous mycobacteriosis is a bacterial disease that is widespread worldwide and characterized by symptoms such as physical weariness, anorexia, and fever. Tuberculosis and nontuberculous mycobacteriosis are known to be caused by the genus *Mycobacterium,* which is a type of *mycobacterium.* Tuberculosis is caused by *Mycobacterium tuberculosis,* and nontuberculous mycobacteriosis is mainly caused by *Mycobacterium avium* and *Mycobacterium intracellulare.*

In addition, since the therapeutic agents and the like are different between tuberculosis and nontuberculous mycobacteriosis, it is clinically important to distinguish tuberculosis from nontuberculous mycobacteriosis.

In the related art, a method of separating and culturing a specimen on a solid medium has been mainly used to distinguish tuberculosis from nontuberculous mycobacteriosis.

However, with this method, it takes approximately 3 weeks for diagnosis of tuberculosis and approximately 4 weeks for diagnosis of nontuberculous mycobacteriosis and is thus impossible to rapidly distinguish tuberculosis from nontuberculous mycobacteriosis.

Therefore, in recent years, with the development of molecular genetics, methods that enable rapid diagnosis using nucleic acid amplification methods such as polymerase chain reaction (PCR) have been developed (Patent Literatures 1 to 3).

The methods of Patent Literatures 1 to 3 are methods for respectively detecting presence or absence of *Mycobacterium tuberculosis,* presence or absence of *Mycobacterium avium*, and presence or absence of *Mycobacterium intracellulare.*

However, since mycobacteriosis such as tuberculosis or nontuberculous mycobacteriosis is caused by *Mycobacterium tuberculosis, Mycobacterium avium,* and *Mycobacterium intracellulare* as described above, it is desirable to simultaneously detect the presence or absence of these three bacterial species from the viewpoint of performing an appropriate treatment.

A method of using primers that anneal to sequences that are commonly conserved in the genus Mycobacterium and using probes that respectively hybridize to sequences specific to *Mycobacterium tuberculosis, Mycobacterium avium,* and *Mycobacterium intracellulare* is known (Patent Literature 4) as a method of simultaneously detecting the presence or absence of *Mycobacterium tuberculosis, Mycobacterium avium,* and *Mycobacterium intracellulare.*

### Citation List

### Patent Literature

[Patent Literature 1] JP4905130B
[Patent Literature 2] JP6166806B
[Patent Literature 3] JP5958498B
[Patent Literature 4] JP1994-261757A (JP-H6-261757A)

### Summary of Invention

### Technical Problem

However, the method of Patent Literature 4 has a problem that the genus *Mycobacterium* present in a sample derived from a subject cannot be appropriately detected, for example, in a case where a difference in the concentration (difference in the number of copies) is found between species of the genus *Mycobacterium* contained in the sample. Specifically, for example, in a case where *Mycobacterium tuberculosis* is present in the sample at a low concentration (for example, 4 copies/µL) and *Mycobacterium avium* and *Mycobacterium intracellulare* are present in the sample at a high concentration (for example, 400 copies/µL), since the amplification amount of the nucleic acid derived from *Mycobacterium tuberculosis* present in the sample at the lowest concentration is extremely less than the amplification amount of the nucleic acid derived from *Mycobacterium avium* and *Mycobacterium intracellulare* present in the sample at the highest concentration, *Mycobacterium tuberculosis* is difficult to detect.

The problem described above is due to the properties of the primer used in the method of Patent Literature 4. That is, since the primer is designed to anneal to a sequence that is commonly conserved in the genus *Mycobacterium,* the primer preferentially anneals to the nucleic acid derived from *Mycobacterium avium* and *Mycobacterium intracellulare,* which are present at the highest concentration in the sample, and as a result, the nucleic acid is preferentially amplified. Therefore, in the method of Patent Literature 4, *Mycobacterium tuberculosis* is difficult to detect even though *Mycobacterium tuberculosis* is present in the sample.

Under the circumstances described above, it is desirable to develop a method that enables detection of the genus *Mycobacterium* (*Mycobacterium tuberculosis*, *Mycobacterium avium,* and *Mycobacterium intracellulare)* present in a sample even in a case where a difference in concentration (number of copies) is found between the species of the genus *Mycobacterium.*

An object of the present invention is to provide a method that enables detection of the genus *Mycobacterium* (*Mycobacterium tuberculosis*, *Mycobacterium avium*, and *Mycobacterium intracellulare)* present in a sample even in a case where a difference in concentration (number of copies) is found between the species of the genus *Mycobacterium.*

### Solution to Problem

The present invention has been made for the purpose of solving the problems, and has the following configurations.
[1] A primer set comprising:
   (i) a primer pair for detecting *Mycobacterium tuberculosis* which consists of a combination of a forward primer consisting of a base sequence represented by SEQ ID NO: 1 and a reverse primer consisting of a base sequence represented by SEQ ID NO: 2;
   (ii) a primer pair for detecting *Mycobacterium avium* which consists of a combination of a forward primer consisting of a base sequence represented by SEQ ID NO: 3 and a reverse primer consisting of a base sequence represented by SEQ ID NO: 4; and
   (iii) a primer pair for detecting *Mycobacterium intracellulare* which consists of a combination of a forward primer consisting of a base sequence represented by SEQ ID NO: 5 and a reverse primer consisting of a base sequence represented by SEQ ID NO: 6.
[2] The primer set according to [1], in which at least one of the forward primers or the reverse primers of the primer pairs (i), (ii) and (iii) are respectively labeled with a labeling substance.
[3] The primer pair according to [1] or [2], in which the labeling substance is selected from a fluorescent substance, a radioactive isotope, or an enzyme.
[4] A method of detecting *Mycobacterium tuberculosis, Mycobacterium avium,* and/or *Mycobacterium intracellulare*, comprising: carrying out a nucleic acid amplification reaction using a nucleic acid in a sample as a template using the primer set according to any one of [1] to [3]; and detecting an obtained nucleic acid amplification product.
[5] A reagent kit comprising:
   (i) a primer pair for detecting *Mycobacterium tuberculosis* which consists of a combination of a forward primer consisting of a base sequence represented by SEQ ID NO: 1 and a reverse primer consisting of a base sequence represented by SEQ ID NO: 2;
   (ii) a primer pair for detecting *Mycobacterium avium* which consists of a combination of a forward primer consisting of a base sequence represented by SEQ ID NO: 3 and a reverse primer consisting of a base sequence represented by SEQ ID NO: 4; and
   (iii) a primer pair for detecting *Mycobacterium intracellulare* which consists of a combination of a forward primer consisting of a base sequence represented by SEQ ID NO: 5 and a reverse primer consisting of a base sequence represented by SEQ ID NO: 6.
[6] The reagent kit according to [5], in which at least one of the forward primers or the reverse primers of the primer pairs (i), (ii) and (iii) are respectively labeled with a labeling substance.

### Advantageous Effects of Invention

According to the primer set for detecting *Mycobacterium tuberculosis, Mycobacterium avium,* and *Mycobacterium intracellulare* of the present invention, the method using the same, and the reagent kit therefor, it is possible to detect the genus Mycobacterium (*Mycobacterium tuberculosis, Mycobacterium avium,* and *Mycobacterium intracellulare*) present in a sample even in a case where a difference in concentration (number of copies) is found between the species of the genus *Mycobacterium.*

### (Brief Description of Drawings)

Fig. 1 shows a detection result of Example 1, obtained by carrying out a nucleic acid amplification reaction (PCR) using various primer sets in the absence of a target.
Fig. 2 shows a detection result of Example 2, obtained by carrying out a nucleic acid amplification reaction (PCR) using a primer set 3 in the presence of a target.
Fig. 3 shows a detection result of Comparative Example 1 (Comparative Example 1-1), obtained by carrying out a nucleic acid amplification reaction (PCR) using a known primer pair and a known probe in the presence of a target.
Fig. 4 shows a detection result of Comparative Example 1 (Comparative Example 1-2), obtained by carrying out a nucleic acid amplification reaction (PCR) using a known primer pair and a known probe in the presence of a target.
Fig. 5 shows a detection result of Comparative Example 1 (Comparative Example 1-3), obtained by carrying out a nucleic acid amplification reaction (PCR) using a known primer pair and a known probe in the presence of a target.
Fig. 6 shows a detection result of Comparative Example 1 (Comparative Example 1-4), obtained by carrying out a nucleic acid amplification reaction (PCR) using a known primer pair and a known probe in the presence of a target.
Fig. 7 shows a detection result of Comparative Example 1 (Comparative Example 1-5), obtained by carrying out a nucleic acid amplification reaction (PCR) using a known primer pair and a known probe in the presence of a target.
Fig. 8 shows a detection result of Comparative Example 1 (Comparative Example 1-6), obtained by carrying out a nucleic acid amplification reaction (PCR) using a known primer pair and a known probe in the presence of a target.
Fig. 9 shows a detection result of Comparative Example 1 (Comparative Example 1-7), obtained by carrying out a nucleic acid amplification reaction (PCR) using a known primer pair and a known probe in the presence of a target.
Fig. 10 shows a test result for the optimum concentration of a primer pair for detecting *Mycobacterium tuberculosis* constituting the primer set 3 in Example 3.
Fig. 11 shows a test result for the optimum concentration of a primer pair for detecting *Mycobacterium avium* constituting the primer set 3 in Example 3.
Fig. 12 shows a test result for the optimum concentration of a primer pair for detecting *Mycobacterium intracellulare* constituting the primer set 3 in Example 3.

### Description of Embodiments

### Primer set for detecting Mycobacterium tuberculosis, Mycobacterium avium, and Mycobacterium intracellulare of present invention

A primer set for detecting *Mycobacterium tuberculosis, Mycobacterium avium,* and *Mycobacterium intracellulare* of the present invention (hereinafter, may be referred to as the primer set of the present invention) contains (i) a primer pair for detecting *Mycobacterium tuberculosis* which consists of a combination of a forward primer consisting of a base sequence represented by SEQ ID NO: 1 and a reverse primer consisting of a base sequence represented by SEQ ID NO: 2 (hereinafter, also referred to as a primer pair for detecting tuberculosis according to the present invention), (ii) a primer pair for detecting *Mycobacterium avium* which consists of a combination of a forward primer consisting of a base sequence represented by SEQ ID NO: 3 and a reverse primer consisting of a base sequence represented by SEQ ID NO: 4 (hereinafter, also referred to as a primer pair for detecting avium according to the present invention), and (iii) a primer pair for detecting *Mycobacterium intracellulare* which consists of a combination of a forward primer consisting of a base sequence represented by SEQ ID NO: 5 and a reverse primer consisting of a base sequence represented by SEQ ID NO: 6 (hereinafter, also referred to as a primer pair for detecting intracellulare according to the present invention).

### Primer pair for detecting tuberculosis according to present invention

The primer pair for detecting tuberculosis according to the present invention consists of a combination of a forward primer consisting of a base sequence represented by SEQ ID NO: 1 and a reverse primer consisting of a base sequence represented by SEQ ID NO: 2.

The forward primer consisting of the base sequence represented by SEQ ID NO: 1 (GenbankID: CP023640.1, base numbers 889765 to 889784) and the reverse primer consisting of the base sequence represented by SEQ ID NO: 2 (GenbankID: CP023640.1, base numbers 889925 to 889946) respectively anneal to the base sequence represented by SEQ ID NO: 7 (GenbankID: CP023640.1, base numbers 889765 to 889946) among the base sequences of *Mycobacterium tuberculosis.*

Therefore, in a case where the nucleic acid amplification reaction such as PCR is carried out using the primer pair for detecting tuberculosis according to the present invention and a nucleic acid-containing sample such as DNA derived from *Mycobacterium tuberculosis,* the base sequence represented by SEQ ID NO: 7 is amplified.

### Primer pair for detecting avium according to present invention

The primer pair for detecting avium according to the present invention consists of a combination of a forward primer consisting of the base sequence represented by SEQ ID NO: 3 and a reverse primer consisting of the base sequence represented by SEQ ID NO: 4.

The forward primer consisting of the base sequence represented by SEQ ID NO: 3 (GenbankID: CP000479.1, base numbers 829817 to 829835) and the reverse primer consisting of the base sequence represented by SEQ ID NO: 4 (GenbankID: CP000479.1, base numbers 829684 to 829703) respectively anneal to the base sequence represented by SEQ ID NO: 8 (GenbankID: CP000479.1, base numbers 829684 to 829835) among the base sequences of *Mycobacterium avium.*

Therefore, in a case where the nucleic acid amplification reaction such as PCR is carried out using the primer pair for detecting avium according to the present invention and a nucleic acid-containing sample such as DNA derived from *Mycobacterium avium,* the base sequence represented by SEQ ID NO: 8 is amplified.

### Primer pair for detecting intracellulare according to present invention

The primer pair for detecting intracellulare according to the present invention consists of a combination of a forward primer consisting of the base sequence represented by SEQ ID NO: 5 and a reverse primer consisting of the base sequence represented by SEQ ID NO: 6.

The forward primer consisting of the base sequence represented by SEQ ID NO: 5 (GenbankID: CP023149.1, base numbers 3015253 to 3015271) and the reverse primer consisting of the base sequence represented by SEQ ID NO: 6 (GenbankID: CP023149.1, base numbers 3015059 to 3015076) anneal to the base sequence represented by SEQ ID NO: 9 (GenbankID: CP023149.1, base numbers 3015059 to 3015271) among the base sequences of *Mycobacterium intracellulare.*

Therefore, in a case where the nucleic acid amplification reaction such as PCR is carried out using the primer pair for detecting intracellulare according to the present invention and a nucleic acid-containing sample such as DNA derived from *Mycobacterium intracellulare,* the base sequence represented by SEQ ID NO: 9 is amplified.

The concentrations (final concentrations) of the primer pair for detecting tuberculosis, the primer pair for detecting avium, and the primer pair for detecting intracellulare according to the present invention in the primer set of the present invention are as listed in Table 1.

It should be noted that the concentration (final concentration) of each primer pair denotes the concentration of the forward primer and the reverse primer constituting the primer pair in a nucleic acid amplification reaction solution such as PCR. For example, in a case where the concentration of the primer pair is 400 nM, it denotes that the concentration of the forward primer and the concentration of the reverse primer constituting the primer pair in the nucleic acid amplification reaction solution such as PCR are respectively 400 nM. Hereinafter, the same applies to the present specification.

**Table 1**

| | Primer pair for detecting tuberculosis according to present invention | Primer pair for detecting avium according to present invention | Primer pair for detecting *intracellulare* according to present invention |
|---|---|---|---|
| Typical concentration (final concentration) | 200 nM~400 nM | 700 nM~900 nM | 500 nM~700 nM |
| Preferable concentration (final concentration) | 250 nM~350 nM | 750 nM~850 nM | 550 nM~650 nM |
| More preferable concentration (final concentration) | 300 nM | 800 nM | 600 nM |

In addition, the concentration ratios (final concentration ratios) of the primer pair for detecting tuberculosis, the primer pair for detecting avium, and the primer pair for detecting intracellulare according to the present invention in the primer set of the present invention are as listed in Table 2.

It should be noted that Table 2 shows the concentration ratios (final concentration ratios) of the primer pairs in the primer set of the present invention which consist of the primer pair for detecting tuberculosis, the primer pair for detecting avium, and the primer pair for detecting intracellulare according to the present invention.

The concentration ratio (final concentration ratio) denotes the concentration (%) of each primer pair in a case where the concentration of the entire primer set is set to 100%. In addition, in the table below, the term "approximately" denotes a range of ±1% of the specified value. Hereinafter, the same applies to the present specification.

**Table 2**

| | Primer pair for detecting tuberculosis according to present invention | Primer pair for detecting avium according to present invention | Primer pair for detecting intracellulare according to present invention |
|---|---|---|---|
| Typical concentration ratio (final concentration ratio) | 10%~20% | 40%∼50% | 30%∼40% |
| Preferable concentration ratio (final concentration ratio) | Approximately 18% | Approximately 47% | Approximately 35% |

As a method of obtaining primers (hereinafter, also referred to as the primers according to the present invention) constituting the primer pair for detecting tuberculosis, the primer pair for detecting avium, and the primer pair for detecting intracellulare according to the present invention (hereinafter, also referred to as the primer pairs according to the present invention), the primers may be obtained by a method known per se which has been typically performed in the field, and specific examples thereof include a method of preparing the primers by a chemical synthesis method such as a phosphoramidite method and a method of obtaining the primers by a gene manipulation method using a vector or the like. Among these, the method of preparing the primers by a chemical synthesis method is preferable.

It is preferable that the primers according to the present invention are labeled with a labeling substance. Specifically, for example, it is preferable that at least one of the forward primers or the reverse primers are labeled with a labeling substance. More specifically, for example, in a case of the primer pair for detecting tuberculosis according to the present invention, at least one of the forward primer consisting of the base sequence represented by SEQ ID NO: 1 or the reverse primer consisting of the base sequence represented by SEQ ID NO: 2 may be labeled with a labeling substance. In the primer pair for detecting avium according to the present invention, at least one of the forward primer consisting of the base sequence represented by SEQ ID NO: 3 or the reverse primer consisting of the base sequence represented by SEQ ID NO: 4 may be labeled with a labeling substance. In addition, in the primer pair for detecting intracellulare according to the present invention, at least one of the forward primer consisting of the base sequence represented by SEQ ID NO: 5 or the reverse primer consisting of the base sequence represented by SEQ ID NO: 6 may be labeled with a labeling substance.

The labeling substance used for labeling the primers according to the present invention is not limited as long as the labeling substance is a substance known per se, which has been typically used in the field, and specific examples thereof include a fluorescent substance, a radioactive isotope, and an enzyme. Among these, the fluorescent substance is preferable.

Examples of the fluorescent substance include TAMRA^{™} (manufactured by Sigma-Aldrich Co. LLC), Alexa555, Alexa647 (manufactured by Thermo Fisher Scientific Inc.), Cyanine Dye-based Cy3, Cy5 (manufactured by GE Healthcare), and fluorescein. Among these, TAMRA^{™} is preferable.

Examples of the radioactive isotope include ³²P, ³³P, and ³⁵S.

Examples of the enzyme include alkaline phosphatase and horseradish peroxidase.

In the primers according to the present invention which have been labeled with the labeling substance, the labeling substance may be bound to the primer directly or via a linker. The linker may be any linker that has been typically used in the field, and specifically, for example, a nucleic acid having 1 to 3 bases is preferable, a DNA having 1 to 3 bases is more preferable, a DNA having 2 bases is still more preferable, and two bases of adenine (A)-adenine (A) are particularly preferable.

As a method of labeling the primers according to the present invention with a fluorescent substance, the primers may be labeled by a method known per se which has been typically used in the field, and specific examples thereof include a method of incorporating a nucleotide labeled with fluorescein into a primer using a method known per se.

As a method of labeling the primers according to the present invention with a radioactive isotope, the primers may be labeled by a method known per se which has been typically used in the field, and specific examples thereof include a method of incorporating a nucleotide labeled with a radioactive isotope into a primer. Specific examples thereof include a random primer method, nick translation, a 5' terminal labeling method using a T4 polynucleotide kinase, and a 3' terminal labeling method using a terminal deoxynucleotidyl transferase.

The primer according to the present invention may be labeled with an enzyme by a method known per se, which has been typically performed in the field, and specific examples thereof include a direct labeling method of covalently binding an enzyme molecule such as alkaline phosphatase or horseradish peroxidase directly to the primer to be labeled.

### Method of detecting Mycobacterium tuberculosis, Mycobacterium avium, and/or Mycobacterium intracellulare of present invention

The method for detecting *Mycobacterium tuberculosis, Mycobacterium avium,* and/or *Mycobacterium intracellulare* of the present invention (hereinafter, also referred to as the detection method of the present invention) is carried out by performing a nucleic acid amplification reaction using a nucleic acid in a sample as a template (hereinafter, also referred to as an amplification step according to the present invention) and detecting the obtained amplification product using the primer set of the present invention (hereinafter, also referred to as a detection step according to the present invention).

### Sample according to present invention

The sample according to the present invention may be any biological sample in which *Mycobacterium tuberculosis, Mycobacterium avium,* and/or *Mycobacterium intracellulare* can be present, and specific examples thereof include sputum, saliva, lung lavage fluid, gastric fluid, whole blood, plasma, serum, urine, feces, skin, and pancreatic fluid. Among these, sputum, saliva, lung lavage fluid, and gastric fluid are preferable, and sputum is more preferable.

The sample according to the present invention may be subjected to an operation of concentrating or separating *Mycobacterium tuberculosis, Mycobacterium avium,* and/or *Mycobacterium intracellulare* present in the sample or an operation of extracting or purifying a nucleic acid from *Mycobacterium tuberculosis, Mycobacterium avium,* and/or *Mycobacterium intracellulare* present in the sample, before the sample is provided for the detection method of the present invention.

The concentration and separation of *Mycobacterium tuberculosis, Mycobacterium avium*, and/or *Mycobacterium intracellulare* and the concentration and separation of *Mycobacterium tuberculosis, Mycobacterium avium,* and/or *Mycobacterium intracellulare* present in the sample may be performed by a method known per se which has been typically performed in the field, and specific examples thereof include filtration and centrifugation.

The extraction and purification of a nucleic acid of *Mycobacterium tuberculosis, Mycobacterium avium,* and/or *Mycobacterium intracellulare* may be performed by a method known per se which has been typically performed in the field, and specific examples thereof include a method of destroying the cell wall of *Mycobacterium tuberculosis, Mycobacterium avium,* and/or *Mycobacterium intracellulare* and treating *Mycobacterium tuberculosis, Mycobacterium avium,* and/or *Mycobacterium intracellulare* with phenol and chloroform and a method of treating *Mycobacterium tuberculosis, Mycobacterium avium*, and/or *Mycobacterium intracellulare* with alcohol such as ethanol or isopropanol.

It should be noted that the method of destroying the cell wall of *Mycobacterium tuberculosis, Mycobacterium avium,* and/or *Mycobacterium intracellulare* may be performed by a method known per se which has been typically performed in the field, and specific examples thereof include a method of using a surfactant such as SDS or a protein denaturing agent such as guanidine thiocyanate and a method of physically crushing the cell wall with glass beads or the like.

### Nucleic acid according to present invention

The nucleic acid according to the present invention denotes a nucleic acid derived from *Mycobacterium tuberculosis, Mycobacterium avium,* and/or *Mycobacterium intracellulare* present in the sample, which is DNA or RNA and preferably DNA.

It should be noted that in a case where the nucleic acid is RNA, complementary DNA (cDNA) may be synthesized by a reverse transcription reaction and subjected to an amplification step according to the present invention described below.

### Amplification step according to present invention

The amplification step according to the present invention may be performed based on a nucleic acid amplification reaction known per se, which has been typically performed in the field, and specific examples thereof include a polymerase chain reaction (PCR) method, a transcription-mediated amplification (TMA) method, and a strand displacement amplification (SDA) method. Among these, the PCR method is preferable.

As the reagent used in the amplification step according to the present invention, any reagent known per se which has been typically used in the field may be used, and specific examples thereof include a nucleic acid synthase such as Taq polymerase, a nucleic acid synthesis substrate such as dNTP, a buffer solution such as a Tris buffer solution or a TAPS buffer solution, and a salt such as MgCl₂, KCl, or (NH₄)₂SO₄.

Further, examples of the reagent include, in addition to the reagents described above, polyethylene glycol, a surfactant such as Triton (manufactured by The Dow Chemical Company), Nonidet (manufactured by Shell Chemicals), CHAPS (manufactured by Dojindo Laboratories), or Tween, a preservative such as Proclin, and a polypeptide such as BSA (bovine serum albumin).

In the amplification step according to the present invention, the amplification reaction may be carried out using a primer pair for detecting an internal control in addition to the primer pair of the present invention.

The internal control may be any bacterium other than *Mycobacterium tuberculosis, Mycobacterium avium,* and *Mycobacterium intracellulare*, and examples thereof include bacteria such as Bacillus subtilis, Bacillus cereus, and Clostridium difficile. Among these, Bacillus subtilis is preferable. It is preferable that the primer pair for detecting Bacillus subtilis described above consists of a combination of a forward primer consisting of the base sequence represented by SEQ ID NO: 21 and a reverse primer consisting of the base sequence represented by SEQ ID NO: 22.

In addition, each of the primers constituting the primer pair for detecting the bacterium selected as the internal control may be labeled with a labeling substance. Specifically, for example, at least one of the forward primer or the reverse primer in the primer pair may be labeled with a labeling substance.

It should be noted that the labeling substance and the method of labeling a primer with the labeling substance are as described in the section of <Primer set of present invention>, and the specific examples, the preferred examples, and the like are also the same as described above.

### Detection step according to present invention

The detection step according to the present invention may be performed by a method known per se which has been typically performed in the field, and specific examples thereof include an end point method and a real-time method. Among these, the real-time method is preferable.

The end point method is a method of carrying out a nucleic acid amplification reaction using the primer pair of the present invention and separating and detecting the obtained amplification product.

Meanwhile, the real-time method is a method of detecting an amplification product obtained by the nucleic acid amplification reaction using the primer pair of the present invention in a real time during the nucleic acid amplification reaction.

Specific examples of the end point method and the real-time method include a labeled primer method (a), an intercalator method (b), and a labeled probe method (c). Among these, the labeled primer method (a) is preferable.

### Labeled primer method (a)

The labeled primer method (a) is, for example, performed in the following manner.

[The nucleic acid amplification reaction is carried out using the nucleic acid in the sample as a template with the primer set of the present invention, in which at least one of each primer pair of the present invention is labeled with a labeling substance. Next, (i) the obtained amplification product is separated after the nucleic acid amplification reaction, and the label in the amplification product is detected (end point method) or (ii) the label in the amplification product obtained every time the nucleic acid amplification reaction is carried out for one to three cycles is detected in a real time (real-time method).

As a result, (i) in a case where fluorescence derived from the label of the amplification product amplified by the primer pair for detecting tuberculosis according to the present invention is detected, it is determined that "the sample is positive for *Mycobacterium tuberculosis."*
(ii) In a case where fluorescence derived from the label of the amplification product amplified by the primer pair for detecting avium according to the present invention is detected, it is determined that "the sample is positive for *Mycobacterium avium."*
(iii) In a case where fluorescence derived from the label of the amplification product amplified by the primer pair for detecting intracellulare according to the present invention is detected, it is determined that "the sample is positive for *Mycobacterium intracellulare.*"]

As the method of "detecting the label in real time" in the labeled primer method, the fluorescence derived from the label of the amplification product may be detected after the amplification product obtained every time the nucleic acid amplification reaction is carried out for one to three cycles is once separated.

It should be noted that "detecting the label" in the present specification denotes that the labeling substance is directly or indirectly measured based on the properties of the labeling substance.

Specific examples of the method for the separation in the labeled primer method (a) include methods known per se, such as electrophoresis and high performance liquid chromatography (HPLC). Among such methods, electrophoresis is preferable.

Specific examples of the electrophoresis include capillary electrophoresis, agarose gel electrophoresis, polyacrylamide gel electrophoresis (slab electrophoresis), starch gel electrophoresis, and isoelectric focusing electrophoresis. Among these, capillary electrophoresis is preferable.

It should be noted that the capillary electrophoresis may be performed, for example, by the methods known per se described in WO2007/027495, WO2011/118496, WO2008/075520, and the like.

### Intercalator method (b)

The intercalator method (b) is, for example, carried out in the following manner in a case of being performed by the end point method.

[The nucleic acid amplification reaction is carried out using the nucleic acid in the sample as a template with the primer set of the present invention. Thereafter, the obtained amplification products are separated. Next, the amplification products are stained with an intercalator, and the fluorescence derived from the intercalator is detected.

As a result, (i) in a case where fluorescence derived from the intercalator in the amplification product amplified by the primer pair for detecting tuberculosis according to the present invention is detected, it is determined that "the sample is positive for *Mycobacterium tuberculosis."*
(ii) In a case where fluorescence derived from the intercalator in the amplification product amplified by the primer pair for detecting avium according to the present invention is detected, it is determined that "the sample is positive for *Mycobacterium avium.*"
(iii) In a case where fluorescence derived from the intercalator in the amplification product amplified by the primer pair for detecting intracellulare according to the present invention is detected, it is determined that "the sample is positive for *Mycobacterium intracellulare.*"]

In addition, the intercalator method (b) is carried out in the following manner in a case of being performed by the real-time method.

[The nucleic acid amplification reaction is carried out using the nucleic acid in the sample as a template with the primer set of the present invention and the intercalator. Next, the fluorescence derived from the intercalator that intercalates in correlation with the amplification amount of the obtained amplification product is detected.

As a result, (i) in a case where an increase in fluorescence derived from the intercalator in the amplification product amplified by the primer pair for detecting tuberculosis according to the present invention is detected, it is determined that "the sample is positive for *Mycobacterium tuberculosis."*
(ii) In a case where an increase in fluorescence derived from the intercalator in the amplification product amplified by the primer pair for detecting avium according to the present invention is detected, it is determined that "the sample is positive for *Mycobacterium avium."*
(iii) In a case where an increase in fluorescence derived from the intercalator in the amplification product amplified by the primer pair for detecting intracellulare according to the present invention is detected, it is determined that "the sample is positive for *Mycobacterium intracellulare.*"]

The separation in the intercalator method is as described in the section of the labeled primer method (a), and the specific examples, the preferred examples, and the like are the same as described above.

The intercalator in the intercalator method may be any intercalator as long as the intercalator is known per se which has been typically used in the field, and specific examples thereof include those described in WO2017/170376. Among the examples, SYTOX (trademark)-based dyes [for example, SYBR Gold (trademark), SYBR Green I (trademark), SYBR Green II (trademark), SYTOX Green (trademark), SYTOX Blue (trademark), and SYTOX Orange (trademark) (all manufactured by Thermo Fisher Scientific Inc.)] are preferable.

### Labeled probe method (c)

The labeled probe method (c) is, for example, carried out in the following manner in a case of being performed by the end point method.

[The nucleic acid amplification reaction is carried out using the nucleic acid in the sample as a template with the primer set of the present invention. Thereafter, the obtained amplification products are separated. Next, the amplification product is treated with a basic solution such as sodium hydroxide to form a single strand. Next, the amplification product is hybridized with a probe labeled with a labeling substance having a base sequence complementary to the base sequence of all or part of the amplification product to form a hybrid material, and the label in the hybrid material is detected.

As a result, (i) in a case where the label derived from the probe hybridized with the amplification product amplified by the primer pair for detecting tuberculosis according to the present invention is detected, it is determined that "the sample is positive for *Mycobacterium tuberculosis."*
(ii) In a case where the label derived from the probe hybridized with the amplification product amplified by the primer pair for detecting avium according to the present invention is detected, it is determined that "the sample is positive for *Mycobacterium avium."*
(iii) In a case where the label derived from the probe hybridized with the amplification product amplified by the primer pair for detecting intracellulare according to the present invention is detected, it is determined that "the sample is positive for *Mycobacterium intracellulare."*]

The labeling substance and the method of performing labeling with the labeling substance in the labeled probe method are as described in the section of <Primer set of present invention>, and the specific examples, the preferred examples, and the like are also the same as described above.

In addition, the labeled probe method (c) is, for example, carried out in the following manner in a case of being performed by the real-time method.

["The nucleic acid amplification reaction is carried out using the nucleic acid in the sample as a template with the primer set of the present invention and the fluorescent labeled probe. Next, the fluorescence derived from the probe in the obtained amplification product is detected.

As a result, (i) in a case where an increase in fluorescence derived from the probe hybridized with the amplification product amplified by the primer pair for detecting tuberculosis according to the present invention is detected, it is determined that "the sample is positive for *Mycobacterium tuberculosis".*
(ii) In a case where an increase in fluorescence derived from the probe hybridized with the amplification product amplified by the primer pair for detecting avium according to the present invention is detected, it is determined that "the sample is positive for *Mycobacterium avium".*
(iii) in a case where an increase in fluorescence derived from the probe hybridized with the amplification product amplified by the primer pair for detecting intracellulare according to the present invention is detected, it is determined that "the sample is positive for *Mycobacterium intracellulare".*]

It should be noted that the fluorescent labeled probe denotes a probe which is designed to hybridize to a region amplified by the nucleic acid amplification reaction using the primer pair according to the present invention and in which the 5' terminal thereof is labeled with, for example, a fluorescent dye (reporter fluorescent dye) and the 3' terminal thereof is labeled with, for example, a quencher dye.

The separation in the labeled probe method is as described in the section of the labeled primer method (a), and the specific examples, the preferred examples, and the like are the same as described above.

### Specific examples of detection method of present invention

Specific examples of the detection method of the present invention will be described below.

First, a nucleic acid (for example, DNA) is extracted from a sample (for example, sputum) by a method known per se.

Next, the primer pair for detecting tuberculosis, the primer pair for detecting avium, and the primer pair for detecting intracellulare according to the present invention are respectively synthesized by the chemical synthesis method (for example, a phosphoramidite method). Thereafter, at least one of each primer pair may be labeled with a labeling substance (for example, a fluorescent substance) by a method known per se.

Next, the nucleic acid amplification reaction (for example, PCR) is carried out in the following manner using the nucleic acid and the primer pair.

That is, 1 to 300 mM of a buffer solution (for example, a Tris buffer solution or a TAPS buffer solution) having a pH of 7 to 10, which contains 200 nM to 400 nM of each primer constituting the primer pair for detecting tuberculosis according to the present invention, 700 nM to 900 nM of each primer constituting the primer pair for detecting avium according to the present invention, 500 nM to 700 nM of each primer constituting the primer pair for detecting intracellulare according to the present invention, 0.5 mM to 5 mM of a salt (for example, MgCl₂), 0.05 mg/mL to 10 mg/mL of a polypeptide (for example, BSA), 0.1 mM to 2 mM of a nucleic acid synthesis substrate (for example, dATP, dCTP, dGT, or dTTP), and 0.5 U/mLto 100 U/mL of a nucleic acid synthase (for example, Taq polymerase), is prepared and used as a solution for a nucleic acid amplification reaction. Next, 0.01 ng to 1000 ng of the nucleic acid is added to 5 µL to 100 µL of the reaction solution, and the resulting reaction solution is used as a sample for a nucleic acid amplification reaction.

Next, the nucleic acid amplification reaction (for example, the PCR method) is carried out by a nucleic acid amplification device such as a thermal cycler under the following conditions.

Condition for nucleic acid amplification reaction (for example, PCR):
After the reaction solution is heated at 93°C to 98°C for 10 seconds to 3 minutes, a cycle of heating at (1) 93°C to 98°C for 1 second to 30 seconds → (2) 50°C to 70°C for 5 seconds to 30 seconds → (3) 60°C to 80°C for 3 to 30 seconds is set as one cycle, and 30 to 50 cycles of heating is performed.

Next, the label in the amplification product obtained every time the nucleic acid amplification reaction is carried out for one to three cycles is detected in a real time.

As a result, (i) in a case where fluorescence derived from the label of the amplification product amplified by the primer pair for detecting tuberculosis according to the present invention is detected, it is determined that "the sample is positive for *Mycobacterium tuberculosis."*
(ii) In a case where fluorescence derived from the label of the amplification product amplified by the primer pair for detecting avium according to the present invention is detected, it is determined that "the sample is positive for *Mycobacterium avium."*
(iii) In a case where fluorescence derived from the label of the amplification product amplified by the primer pair for detecting intracellulare according to the present invention is detected, it is determined that "the sample is positive for *Mycobacterium intracellulare."*

### Reagent kit for detecting Mycobacterium tuberculosis, Mycobacterium avium, and/or Mycobacterium intracellulare of present invention

A reagent kit for detecting *Mycobacterium tuberculosis, Mycobacterium avium*, and/or *Mycobacterium intracellulare* of the present invention (hereinafter, also referred to as the reagent kit of the present invention) contains the primer set of the present invention.

The reagent kit of the present invention is required to contain (A) the primer set of the present invention, but may contain, for example, the following items (B), (C), (D), (E), (F) and/or (G).
(B): Primer pair for detecting internal control
(C): Sterilizing liquid
(D): Nucleic acid extraction-related reagent
(E): Nucleic acid amplification-related reagent
(F): Electrophoresis-related reagent
(G): Manual

### (B): Primer pair for detecting internal control

(B) The primer pair for detecting an internal control is used to detect bacteria selected as the internal control, and specific examples thereof include a primer pair for detecting Bacillus subtilis, Bacillus cereus, and Clostridium difficile. Among the examples, a primer pair for detecting Bacillus subtilis is preferable, and a primer pair consisting of a forward primer consisting of the base sequence represented by SEQ ID NO: 21 and a reverse primer consisting of the base sequence represented by SEQ ID NO: 22 is preferable.

### (C): Sterilizing liquid

(C) The sterilizing liquid is a liquid for sterilizing the genus *Mycobacterium* (*Mycobacterium tuberculosis*, *Mycobacterium avium*, *Mycobacterium intracellulare*, and the like) present in the sample and bacteria selected as the internal control, and specifically, for example, a sterilizing liquid containing alcohol such as 2-propanol is preferable. In addition, it is preferable that the sterilizing liquid contains the bacteria selected as the internal control.

### (D): Nucleic acid extraction-related reagent

(D) The nucleic acid extraction-related reagent is used to extract a nucleic acid from the genus *Mycobacterium* (*Mycobacterium tuberculosis*, *Mycobacterium avium*, *Mycobacterium intracellulare*, and the like) present in the sample, and examples thereof include a nucleic acid binding solution, a nucleic acid eluate, and a nucleic acid washing solution.

Specifically, a nucleic acid binding solution containing, for example, a protein denaturing agent such as a guanidine hydrochloride, a Tris buffer solution, a Tris/EDTA buffer solution (TE buffer solution), and/or a deoxyribonucleic acid sodium salt derived from salmon testis is preferable as the nucleic acid binding solution.

Specifically, a nucleic acid eluate containing, for example, a buffer solution such as a Tris buffer solution and/or a preservative such as Proclin is preferable as the nucleic acid eluate.

In addition, specifically, a nucleic acid washing solution containing, for example, a salt such as sodium chloride, a buffer solution such as a Tris buffer solution, an alcohol such as ethanol, and/or a surfactant such as Tween is preferable as the nucleic acid washing solution.

### (E): Reagent for nucleic acid amplification reaction

(E) The reagent for a nucleic acid amplification reaction is used to carry out a nucleic acid amplification reaction such as PCR, and specific preferred examples thereof include a buffer solution such as a Tris buffer solution or a TAPS buffer solution, a nucleic acid synthesis substrate, a nucleic acid synthase, a preservative such as Proclin, and/or a polypeptide such as BSA.

### (F): Electrophoresis-related reagent

(F) The electrophoresis-related reagent is used for subjecting a nucleic acid amplification product obtained by the nucleic acid amplification reaction to electrophoresis, and specific examples thereof include a buffer solution for electrophoresis, a polymer solution, and a marker for electrophoresis.

Specific preferred examples of the buffer solution for electrophoresis include a salt such as MgCl₂, a buffer solution such as TAPS, an alcohol such as glycerin, and/or a preservative such as Proclin.

Specific preferred examples of the polymer solution include a solution containing a macromolecular polymer such as polyethylene glycol, a cellulose derivative, agarose, polyacrylamide, or a polymer having a monomer unit derived from dimethylformamide.

Specific preferred examples of the marker for electrophoresis include a nucleic acid (DNA or the like) having a known molecular weight.

### (G): Manual

(G) The manual denotes a description in which the features, the principles, the operating procedures, the determination procedures, and the like of the detection method of the present invention are substantially described by means of sentences, figures, tables, and the like, and specific preferred examples thereof include an instruction manual, an attached document, and a pamphlet of the reagent kit of the present invention.

As the reagent kit of the present invention, it is preferable that the items (A) to (G) described above are accommodated in individual containers (tubes and the like).

### Method of assisting diagnosis of mycobacteriosis according to present invention

A method of assisting diagnosis of mycobacteriosis according to the present invention (hereinafter, also referred to as an assisting method according to the present invention) is performed by determining whether a subject suffers from mycobacteriosis based on the results of the detection method of the present invention (hereinafter, also referred to as a determination step according to the present invention).

### Determination step according to present invention

The determination step according to the present invention is performed, for example, as follows, based on the results of the detection method of the present invention.

That is, (i) in a case where a signal (fluorescence or the like) derived from a product amplified by the primer pair for detecting tuberculosis according to the present invention is detected, it is determined that "the subject derived from the sample is likely to be suffering from mycobacteriosis (tuberculosis) or highly likely to be suffering from mycobacteriosis (tuberculosis)". In addition, (ii) in a case where a signal (fluorescence or the like) derived from a product amplified by the primer pair for detecting tuberculosis according to the present invention is not detected, it is determined that "the subject derived from the sample is not suffering from mycobacteriosis (tuberculosis) or unlikely to be suffering from mycobacteriosis (tuberculosis)".
(i') In a case where a signal (fluorescence or the like) derived from a product amplified by the primer pair for detecting avium according to the present invention is detected, it is determined that "the subject derived from the sample is likely to be suffering from mycobacteriosis (nontuberculous mycobacteriosis) or highly likely to be suffering from mycobacteriosis (nontuberculous mycobacteriosis)". In addition, (ii') in a case where a signal (fluorescence or the like) derived from a product amplified by the primer pair for detecting avium according to the present invention is not detected, it is determined that "the subject derived from the sample is not be suffering from mycobacteriosis (nontuberculous mycobacteriosis) or unlikely to be suffering from mycobacteriosis (nontuberculous mycobacteriosis)".

In addition, (i") in a case where a signal (fluorescence or the like) derived from a product amplified by the primer pair for detecting intracellulare according to the present invention is detected, it is determined that "the subject derived from the sample is likely to be suffering from mycobacteriosis (nontuberculous mycobacteriosis) or highly likely to be suffering from mycobacteriosis (nontuberculous mycobacteriosis)". In addition, (ii") in a case where a signal (fluorescence or the like) derived from a product amplified by the primer pair for detecting intracellulare according to the present invention is not detected, it is determined that "the subject derived from the sample is not suffering from mycobacteriosis (nontuberculous mycobacteriosis) or unlikely to be suffering from mycobacteriosis (nontuberculous mycobacteriosis)".

According to the assisting method according to the present invention, it is possible to diagnose mycobacteriosis (tuberculosis and/or nontuberculous mycobacteriosis) with high accuracy (sensitivity/specificity).

### Method of treating mycobacteriosis according to present invention

A method of treating mycobacteriosis according to the present invention (hereinafter, also referred to as a treatment method according to the present invention) is carried out by performing an appropriate treatment (hereinafter, also referred to as a treatment step according to the present invention) based on the results of the assisting method according to the present invention.

### Treatment step according to present invention

The treatment step according to the present invention will be described separately for a case where it is determined that (i) the subject is likely to be suffering from tuberculosis or highly likely to be suffering from tuberculosis, a case where it is determined that (ii) the subject is likely to be suffering from nontuberculous mycobacteriosis or highly likely to be suffering from nontuberculous mycobacteriosis, and a case where it is determined that (iii) the subject is likely to be suffering from tuberculosis and nontuberculous mycobacteriosis or highly likely to be suffering from tuberculosis and nontuberculous mycobacteriosis.

### Case where it is determined that (i) subject is likely to be suffering from tuberculosis or highly likely to be suffering from tuberculosis

In the case of (i), specific examples of the treatment step according to the present invention include drug therapy carried out by administering antibiotics effective for tuberculosis, such as Rifampicin (generic name), Isoniazid (hydrazide) (generic name), Streptomycin (generic name), Ethambutol (generic name), and Pyrazinamide (generic name), and surgical therapy.

### Case where it is determined that (ii) subject is likely to be suffering from nontuberculous mycobacteriosis or highly likely to be suffering from nontuberculous mycobacteriosis

In the case of (ii), specific examples of the treatment step according to the present invention include drug therapy carried out by administering antibiotics effective for nontuberculous mycobacteriosis, such as Clarithromycin (generic name), Ethambutol (generic name), and Rifampicin (generic name), and surgical therapy.

### Case where it is determined that (iii) subject is likely to be suffering from tuberculosis and nontuberculous mycobacteriosis and highly likely to be suffering from tuberculosis and nontuberculous mycobacteriosis

In the case of (iii), specific examples of the treatment step according to the present invention include drug therapy carried out by respectively administering antibiotics effective for tuberculosis and antibiotics effective for nontuberculous mycobacteriosis, and surgical therapy.

According to the treatment method according to the present invention, an appropriate treatment is performed on the subject that is determined to be likely to suffer from mycobacteriosis (tuberculosis and/or nontuberculous mycobacteriosis) or highly likely to suffer from mycobacteriosis (tuberculosis and/or nontuberculous mycobacteriosis).

### Device for assisting diagnosis of mycobacteriosis according to present invention

A device for assisting diagnosis of mycobacteriosis according to the present invention (hereinafter, also referred to as a device according to the present invention) includes at least a nucleic acid amplification reaction unit (1) and a detection unit (2). The device may further include a nucleic acid extraction unit (3), a determination unit (4), an output unit (5), and an input unit (6).

The nucleic acid amplification reaction unit (1) in the device according to the present invention is configured to perform the nucleic acid amplification reaction using the primer set of the present invention.

The detection unit (2) in the device according to the present invention is configured to detect the nucleic acid amplification product obtained by the nucleic acid amplification reaction unit (1).

It should be noted that the nucleic acid amplification reaction unit (1) and the detection unit (2) may be configured independently or integrally, and specific preferred examples thereof include the microfluidic devices described in JP2018-89611A and JP2015-514994A.

The nucleic acid extraction unit (3) in the device according to the present invention is configured to extract and/or purify the nucleic acid from the sample. Specific preferred examples thereof include those configured such that the nucleic acid extraction method described in WO2016/079981 and the nucleic acid purification method described in WO2015/157650 can be carried out.

The determination unit (4) of the device according to the present invention is configured such that it can be determined whether the subject is suffering from mycobacteriosis (tuberculosis and/or nontuberculous mycobacteriosis) based on the results obtained by the detection unit (2).

The output unit (5) in the device according to the present invention is configured to output the result obtained by the nucleic acid amplification reaction unit (1), the detection unit (2), the nucleic acid extraction unit (3), and/or the determination unit (4).

The input unit (6) in the device according to the present invention is configured to send a signal for operating the nucleic acid amplification reaction unit (1) and/or the nucleic acid extraction unit (3) to the nucleic acid amplification reaction unit (1) and/or the nucleic acid extraction unit (3).

According to the device according to the present invention, the assisting method according to the present invention can be easily performed in a short time with high accuracy.

Hereinafter, the present invention will be described in detail based on the following examples, but the present invention is not limited to such examples.

### Examples

### Example 1. Examination of primer set in absence of target

Various primer sets were examined by performing a nucleic acid amplification reaction using various primer sets in the absence of a target.

### (1) Primer set

The primer sets listed in Table 3 were used.

**Table 3**

| Primer set | Target | Primer pair | | Amplification region |
|---|---|---|---|---|
| Primer set 1 | Mycobacterium tuberculosis | Forward primer | SEQ ID NO: 10 | SEQ ID NO: 14 (Base pair at position 178) |
| | | Reverse primer | SEQ ID NO: 11 | |
| | Mycobacterium avium | Forward primer | SEQ ID NO: 3 | SEQ ID NO: 8 (Base pair at position 152) |
| | | Reverse primer | SEQ ID NO: 4 | |
| | Mycobacterium intracellulare | Forward primer | SEQ ID NO: 5 | SEQ ID NO: 9 (Base pair at position 213) |
| | | Reverse primer | SEQ ID NO: 6 | |
| Primer set 2 | Mycobacterium tuberculosis | Forward primer | SEQ ID NO: 10 | SEQ ID NO: 14 (Base pair at position 178) |
| | | Reverse primer | SEQ ID NO: 11 | |
| | Mycobacterium avium | Forward primer | SEQ ID NO: 12 | SEQ ID NO: 15 (Base pair at position 145) |
| | | Reverse primer | SEQ ID NO: 13 | |
| | Mycobacterium intracellulare | Forward primer | SEQ ID NO: 5 | SEQ ID NO: 9 (Base pair at position 213) |
| | | Reverse primer | SEQ ID NO: 6 | |
| Primer set 3 | Mycobacterium tuberculosis | Forward primer | SEQ ID NO: 1 | SEQ ID NO: 7 (Base pair at position 182) |
| | | Reverse primer | SEQ ID NO: 2 | |
| | Mycobacterium avium | Forward primer | SEQ ID NO: 3 | SEQ ID NO: 8 (Base pair at position 152) |
| | | Reverse primer | SEQ ID NO: 4 | |
| | Mycobacterium intracellulare | Forward primer | SEQ ID NO: 5 | SEQ ID NO: 9 (Base pair at position 213) |
| | | Reverse primer | SEQ ID NO: 6 | |
| Primer set 4 | Mycobacterium tuberculosis | Forward primer | SEQ ID NO: 1 | SEQ ID NO: 7 (Base pair at position 182) |
| | | Reverse primer | SEQ ID NO: 2 | |
| | Mycobacterium avium | Forward primer | SEQ ID NO: 12 | SEQ ID NO: 15 (Base pair at position 145) |
| | | Reverse primer | SEQ ID NO: 13 | |
| | Mycobacterium intracellulare | Forward primer | SEQ ID NO: 5 | SEQ ID NO: 9 (Base pair at position 213) |
| | | Reverse primer | SEQ ID NO: 6 | |

### (2) Preparation of sample (DNA)

10 mg/mL of salmon sperm-derived deoxyribonucleic acid (manufactured by Sigma-Aldrich Co. LLC) was prepared using a TE buffer solution (pH of 8.0) (manufactured by Nippon Gene Co., Ltd.), and this deoxyribonucleic acid was used as a sample (DNA).

### (3) Nucleic acid amplification reaction/detection

A 10 mM Tris-HCl buffer solution (pH of 8.0) containing 400 nM of each primer in the primer set 1, 1.5 mM of MgCl₂, 0.5 mg/mL of BSA, 0.2 mM of dATP, 0.2 mM of dCTP, 0.2 mM of dGTP, 0.2 mM of dTTP, and 60 U/mL of KOD Exo (-) (manufactured by Toyobo Co., Ltd.) was prepared and used as a reaction solution for PCR.

In addition, a reaction solution for PCR was prepared in the same manner as described above except for using each primer in the primer set 2, 3, or 4 in place of each primer in the primer set 1.

Next, the DNA prepared in the section (2) was suspended in 25 µL of the reaction solution for PCR such that the final amount thereof reached 5 µg and was used as a sample for PCR.

Each sample for PCR was placed in a 96-well plate (manufactured by Thermo Fisher Scientific Inc.), and PCR was performed using StepOnePlus^{™} (manufactured by Thermo Fisher Scientific Inc.).

It should be noted that PCR was carried out such that the sample was heated at 97°C for 30 seconds, a cycle of heating at (1) 97°C for 10 seconds → (2) 65°C for 20 seconds → (3) 72°C for 30 seconds was set as one cycle, and 40 cycles of heating was performed.

After completion of PCR, the obtained amplification product was separated and detected by performing capillary electrophoresis on each of the obtained nucleic acid amplification product using a fully automatic microchip type capillary electrophoresis device 2100 bioanalyzer (manufactured by Agilent Technologies Japan, Ltd.) according to the protocol attached to the device.

### (4) Results

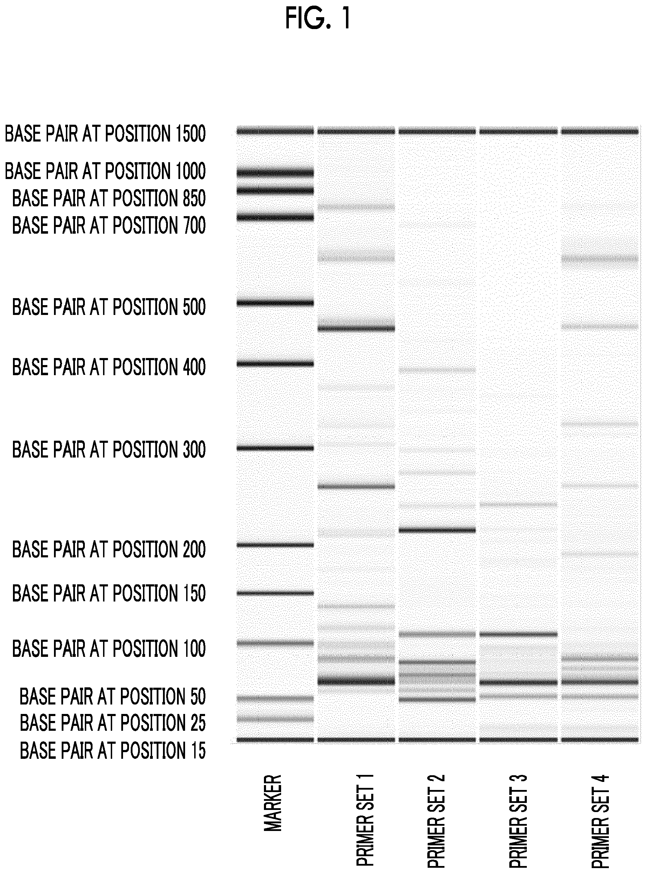

The results obtained in the section (3) are shown in Fig. 1.

It should be noted that each band in Fig. 1 indicates that the nucleic acid amplification product was detected.

In addition, the sample for PCR did not contain DNA derived from *Mycobacterium tuberculosis, Mycobacterium avium,* and *Mycobacterium intracellulare.* Therefore, in a case of detecting the band indicating that the nucleic acid amplification product was detected, this indicates that the nucleic acid was amplified non-specifically (a non-specific nucleic acid amplification product was obtained).

As is evident in Fig. 1, a plurality of high-intensity (dense) bands indicating non-specific nucleic acid amplification were detected in the primer set 1, the primer set 2, and the primer set 4.

In particular, it was found that since the band present in the vicinity of the base pair at position 150 seen in the primer set 1 is extremely close to the position of the product amplified by the primer pair for detecting *Mycobacterium avium,* the possibility of false positives is extremely high. In addition, it was found that since the band present in the vicinity of the base pair at position 200 seen in the primer set 2 is extremely close to the position of the product amplified by the primer pair for detecting *Mycobacterium intracellulare,* the possibility of false positives is extremely high. Further, it was found that since the band present in the vicinity of the base pair at position 180 seen in the primer set 4 is extremely close to the position of the product amplified by the primer pair for detecting *Mycobacterium tuberculosis,* the possibility of false positives is extremely high.

On the contrary, a band showing non-specific amplification of the nucleic acid was not detected in the primer set 3, and thus, no band was present at positions (in the vicinity of the base pair at position 150 to the base pair at position 200) of the product amplified by the primer pair for detecting *Mycobacterium tuberculosis,* the primer pair for detecting *Mycobacterium avium,* and the primer pair for detecting *Mycobacterium intracellulare.*

As shown in the results described above, it was found that since the primer set 3 can suppress non-specific amplification of the nucleic acid, the possibility of false positives is extremely low.

### Example 2. Detection of Mycobacterium tuberculosis, Mycobacterium avium, and Mycobacterium intracellulare

*Mycobacterium tuberculosis, Mycobacterium avium,* and *Mycobacterium intracellulare* were detected using the primer set 3 in Example 1.

### (1) Primer set

The primer set 3 of Example 1 was used.

### (2) Preparation of template DNA

*Mycobacterium tuberculosis* var.BCG, *Mycobacterium avium,* and *Mycobacterium intracellulare* were cultured by a method known per se to obtain purified genomic DNA using a nucleic acid purification method known per se. Next, the number of copies of each purified DNA was calculated by a real-time PCR device (StepOnePlus^{™} (manufactured by Thermo Fisher Scientific Inc.)) using SYBR^{™} Premix Ex TaqTM II (manufactured by Takara Bio Inc.). Thereafter, the purified DNA was prepared as *Mycobacterium tuberculosis* DNA, *Mycobacterium avium* DNA, and *Mycobacterium intracellulare* DNA using a TE buffer solution (pH of 8.0) (manufactured by Nippon Gene Co., Ltd.).

### (3) Nucleic acid amplification reaction/detection

A buffer solution containing the primer pair for detecting *Mycobacterium tuberculosis* (300 nM of each primer), the primer pair for detecting *Mycobacterium avium* (800 nM of each primer), the primer pair for detecting *Mycobacterium intracellulare* (600 nM of each primer), 1.0 mM of MgCl₂, 0.2 mg/mL of BSA, 0.4 mM of dATP, 0.4 mM of dCTP, 0.4 mM of dGTP, 0.4 mM of dTTP, and 1U/mL of Takara Ex Taq (manufactured by Takara Bio Inc.) was prepared and used as a reaction solution for PCR.

The template DNA prepared in the section (3) was suspended in 25 µL of the reaction solution for PCR such that the concentration thereof was set as listed in Table 4 and was used as a sample for PCR.

**Table 4**

| | Concentration of Mycobacterium tuberculosis (number of copies) | Concentration of Mycobacterium avium (number of copies) | Concentration of Mycobacterium intracellulare (number of copies) |
|---|---|---|---|
| Example 2-1 | 400 copies/µL | 400 copies/µL | 400 copies/µL |
| Example 2-2 | 400 copies/µL | 400 copies/µL | 4 copies/µL |
| Example 2-3 | 400 copies/µL | 4 copies/µL | 400 copies/µL |
| Example 2-4 | 4 copies/µL | 400 copies/µL | 400 copies/µL |
| Example 2-5 | 400 copies/µL | 4 copies/µL | 4 copies/µL |
| Example 2-6 | 4 copies/µL | 400 copies/µL | 4 copies/µL |
| Example 2-7 | 4 copies/µL | 4 copies/µL | 400 copies/µL |
| Example 2-8 | 0 copies/µL | 0 copies/µL | 0 copies/µL |

The sample for PCR was placed in a 96-well plate (manufactured by Thermo Fisher Scientific Inc.), and PCR was performed using StepOnePlus^{™} (manufactured by Thermo Fisher Scientific Inc.). The reaction was carried out such that the sample was heated at 97°C for 30 seconds, a cycle of heating at (1) 97°C for 10 seconds → (2) 64°C for 10 seconds → (3) 72°C for 20 seconds was set as one cycle, and 40 cycles of heating was performed.

After completion of PCR, the obtained nucleic acid amplification product was separated and detected by performing capillary electrophoresis on the obtained nucleic acid amplification product using a fully automatic microchip type capillary electrophoresis device 2100 bioanalyzer (manufactured by Agilent Technologies Japan, Ltd.) according to the protocol attached to the device.

### (4) Results

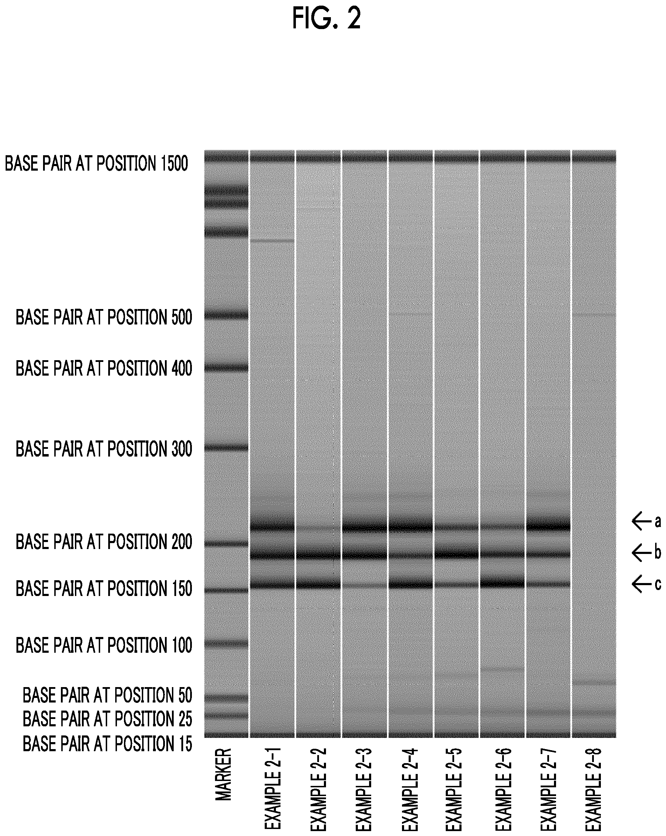

The results of the obtained electrophoresis are shown in Fig. 2.

It should be noted that each band in Fig. 2 indicates that the nucleic acid amplification product was detected.

As is evident in Fig. 2, in all cases of Examples 2-1 to 2-7, the band (band a) of the product (base pair at position 182) amplified by the primer pair for detecting *Mycobacterium tuberculosis,* the band (band b) of the product (base pair at position 152) amplified by the primer pair for detecting *Mycobacterium avium,* and the band (band c) of the product (base pair at position 213) amplified by the primer pair for detecting *Mycobacterium intracellulare* were respectively detected.

In particular, the bands of the amplification products were respectively detected even in a case where a difference in the concentration (number of copies) was found between *Mycobacterium tuberculosis, Mycobacterium avium,* and *Mycobacterium intracellulare* as in Examples 2-2 to 2-7.

Therefore, it was found that *Mycobacterium tuberculosis, Mycobacterium avium,* and *Mycobacterium intracellulare* were able to be detected by using the primer set 3.

Further, it was found that *Mycobacterium tuberculosis, Mycobacterium avium,* and *Mycobacterium intracellulare* were able to be detected even in a case where a difference in the concentration (number of copies) was found between *Mycobacterium tuberculosis, Mycobacterium avium,* and *Mycobacterium intracellulare.*

### Comparative example 1. Detection of Mycobacterium tuberculosis, Mycobacterium avium, and Mycobacterium intracellulare by method of related art

*Mycobacterium tuberculosis, Mycobacterium avium*, and *Mycobacterium intracellulare* were detected by the method of the related art (method of Patent Literature 4).

### (1) Primer set and probe

The primer sets and probes shown in Table 5 were used.

It should be noted that in the probe for detecting *mycobacterium tuberculosis*, the 5' terminal of the probe consisting of the base sequence represented by SEQ ID NO: 18 was labeled with a fluorescent substance (FAM), and the 3' terminal thereof was labeled with a fluorescent substance (TAMRA^{™}).

In the probe for detecting *mycobacterium intracellulare*, the 5' terminal of the probe consisting of the base sequence represented by SEQ ID NO: 19 was labeled with a fluorescent substance (FAM), and the 3' terminal thereof was labeled with a fluorescent substance (TAMRA^{™}).

In addition, in the probe for detecting *mycobacterium avium*, the 5' terminal of the probe consisting of the base sequence represented by SEQ ID NO: 20 was labeled with a fluorescent substance (FAM), and the 3' terminal thereof was labeled with a fluorescent substance (TAMRA^{™}).

**Table 5**

| | Target | Primer pair | | Probe (target sequence) |
|---|---|---|---|---|
| Comparative Example 1 | Mycobacterium tuberculosis | Forward primer | SEQ ID NO: 16 | SEQ ID NO: 18 |
| | | Reverse primer | SEQ ID NO: 17 | |
| | Mycobacterium intracellulare | Forward primer | SEQ ID NO: 16 | SEQ ID NO: 19 |
| | | Reverse primer | SEQ ID NO: 17 | |
| | Mycobacterium avium | Forward primer | SEQ ID NO: 16 | SEQ ID NO: 20 |
| | | Reverse primer | SEQ ID NO: 17 | |

### (2) Preparation of template DNA

*Mycobacterium tuberculosis* DNA, *Mycobacterium avium* DNA, and *Mycobacterium intracellulare* DNA were respectively prepared in the same manner as in the section (2) of Example 2.

### (3) Nucleic acid amplification reaction/detection

A buffer solution containing 500 nM of each primer, 280 nM of each probe, 1.0 mM of MgCl₂, 0.2 mg/mL of BSA, 0.4 mM of dATP, 0.4 mM of dCTP, 0.4 mM of dGTP, 0.4 mM of dTTP, and 1U/mL of Takara Ex Taq (manufactured by Takara Bio Inc.) was prepared and used as a reaction solution for PCR.

The DNA prepared in the section (2) was suspended in 25 µL of the reaction solution for PCR such that the concentration thereof was set as listed in Table 6 and was used as a sample for PCR.

**Table 6**

| | Target | Primer pair | Probe (target sequence) | Concertration of Mycobacterium tuberculosis (number of copies) | Concentration of Mycobacterium avium (number of copies) | Corcentration of Mycobacterium intracellulare (number of copies) |
|---|---|---|---|---|---|---|
| Comparative Example 1-1 | Mycobacterium tuberculosis | Forward primer SEQ ID NO: 16 Reverse primer SEQ ID NO: 17 | SEQ ID NO: 18 | 400 copies/µL | 400 copies/µL | 400 copies/µL |
| | Mycobacterium intracellulare | | SEQ ID NO: 19 | | | |
| | Mycobacterium avium | | SEQ ID NO: 20 | | | |
| Comparative Example 1-2 | Mycobacterium tuberculosis | Forward primer SEQ ID NO: 16 Reverse primer SEQ ID NO: 17 | SEQ ID NO: 18 | 400 copies/µL | 400 copies/µL | 4 copies/µL |
| | Mycobacterium intracellulare | | SEQ ID NO: 19 | | | |
| | Mycobacterium avium | | SEQ ID NO: 20 | | | |
| Comparative Example 1-3 | Mycobacterium tuberculosis | Forward primer SEQ ID NO: 16 Reverse primer SEQ ID NO: 17 | SEQ ID NO: 18 | 400 copies/µL | 4 copies/µL | 400 copies/µL |
| | Mycobacterium intracellulare | | SEQ ID NO: 19 | | | |
| | Mycobacterium avium | | SEQ ID NO: 20 | | | |
| Comparative Example 1-4 | Mycobacterium tuberculosis | Forward primer SEQ ID NO: 16 Reverse primer SEQ ID NO: 17 | SEQ ID NO: 18 | 4 copies/µL | 400 copies/µL | 400 copies/µL |
| | Mycobacterium intracellulare | | SEQ ID NO: 19 | | | |
| | Mycobacterium avium | | SEQ ID NO: 20 | | | |
| Comparative Example 1-5 | Mycobacterium tuberculosis | Forward primer SEQ ID NO: 16 Reverse primer SEQ ID NO: 17 | SEQ ID NO: 18 | 400 copies/µL | 4 copies/µL | 4 copies/µL |
| | Mycobacterium intracellulare | | SEQ ID NO: 19 | | | |
| | Mycobacterium avium | | SEQ ID NO: 20 | | | |
| Comparative Example 1-6 | Mycobacterium tuberculosis | Forward primer SEQ ID NO: 16 Reverse primer SEQ ID NO: 17 | SEQ ID NO: 18 | 4 copies/µL | 400 copies/µL | 4 copies/µL |
| | Mycobacterium intracellulare | | SEQ ID NO: 19 | | | |
| | Mycobacterium avium | | SEQ ID NO: 20 | | | |
| Comparative Example 1-7 | Mycobacterium tuberculosis | Forward primer SEQ ID NO: 16 Reverse primer SEQ ID NO: 17 | SEQ ID NO: 18 | 4 copies/µL | 4 copies/µL | 400 copies/µL |
| | Mycobacterium intracellulare | | SEQ ID NO: 19 | | | |
| | Mycobacterium avium | | SEQ ID NO: 20 | | | |

The sample for PCR was placed in a 96-well plate (manufactured by Thermo Fisher Scientific Inc.), real-time PCR was performed using StepOnePlus^{™} (manufactured by Thermo Fisher Scientific Inc.), and the nucleic acid amplification product was detected in a real time. The reaction was carried out such that the sample was heated at 97°C for 30 seconds, a cycle of heating at (1) 97°C for 10 seconds → (2) 64°C for 10 seconds → (3) 72°C for 20 seconds was set as one cycle, and 40 cycles of heating was performed.

### (4) Results

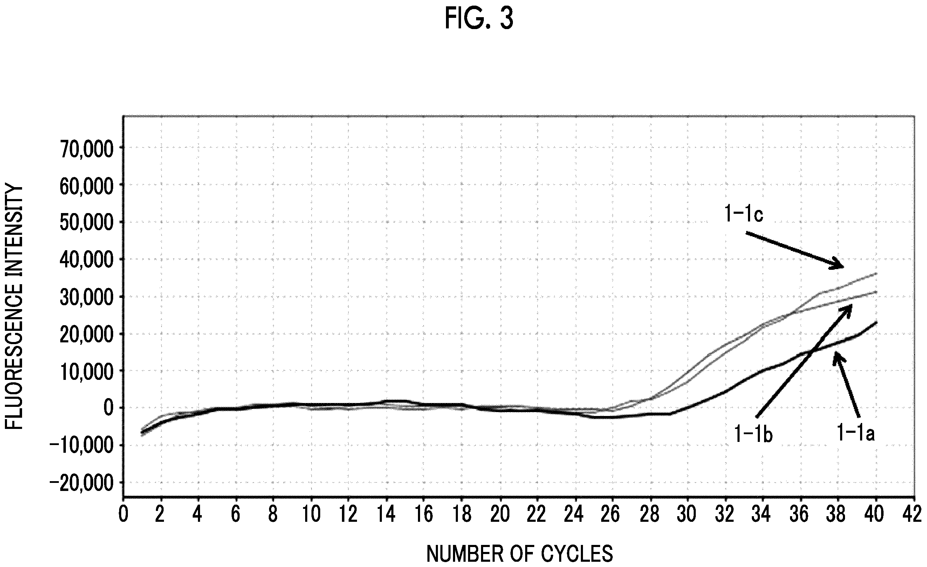

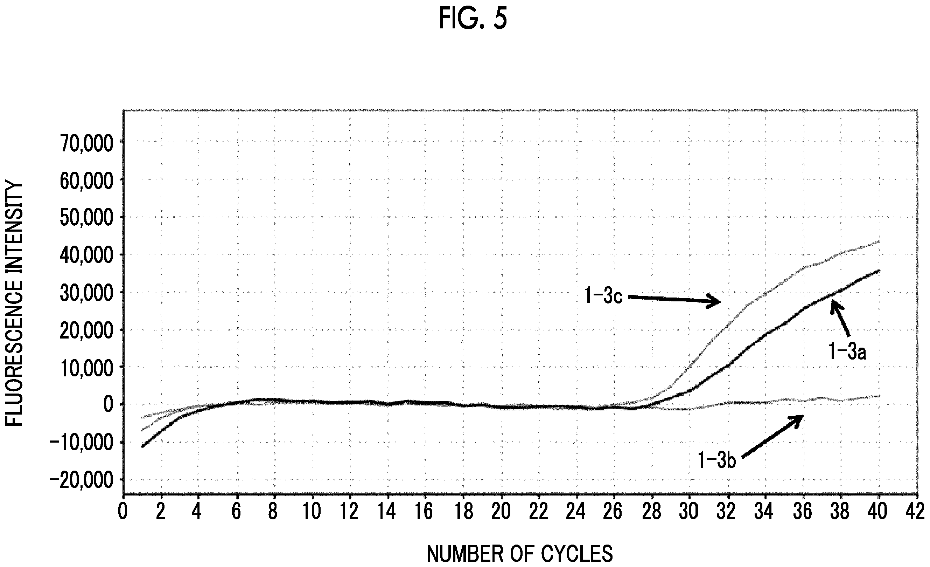

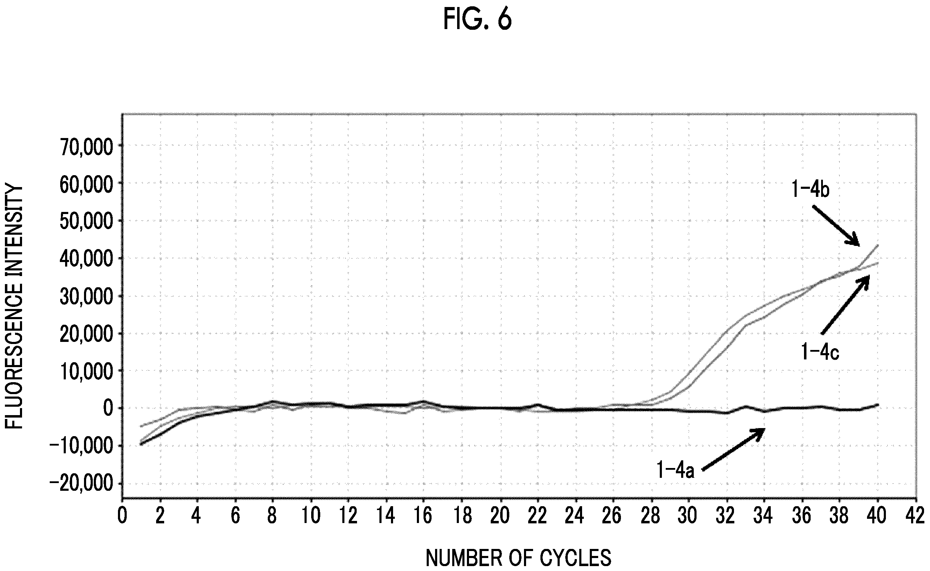

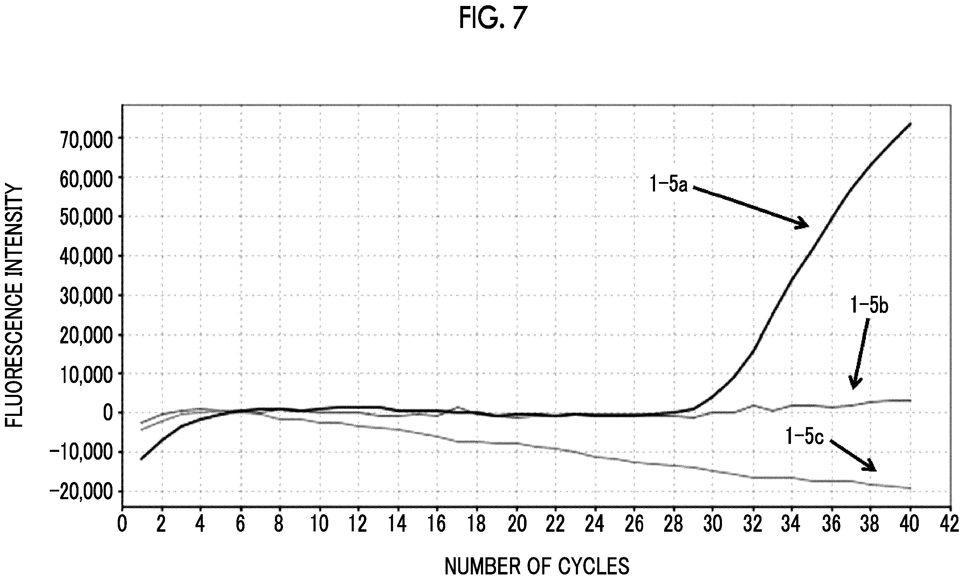

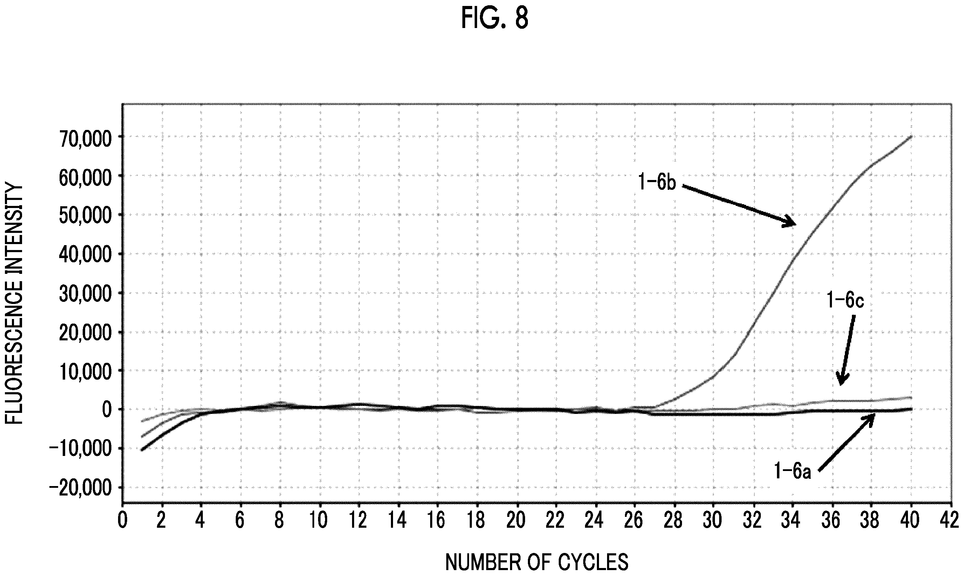

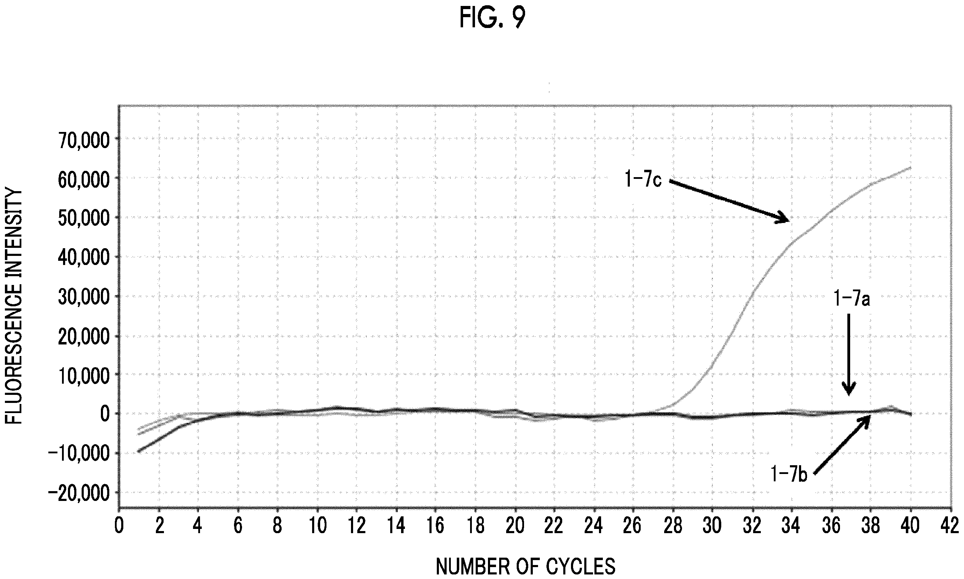

The results of the real-time PCR in the section (3) are respectively shown in Figs. 3 to 9.

It should be noted that Figs. 3 to 9 show that the nucleic acid amplification product whose purpose was to increase the fluorescence intensity was detected. In addition, in Figs. 3 to 9, 1-1a to 1-7a represent the detection results of *Mycobacterium tuberculosis,* 1-1b to 1-7b represent the detection results of *Mycobacterium avium,* and 1-1c to 1-7c represent the detection results of *Mycobacterium intracellulare.*

As is evident in the results of Fig. 3 (Comparative Example 1-1), in a case where the concentrations of *Mycobacterium tuberculosis, Mycobacterium avium,* and *Mycobacterium intracellulare* were the same as each other, an increase in fluorescence intensity was confirmed (1-1a, 1-1b, and 1-1c in Fig. 3). Therefore, *Mycobacterium tuberculosis, Mycobacterium avium*, and *Mycobacterium intracellulare* were able to be respectively detected.

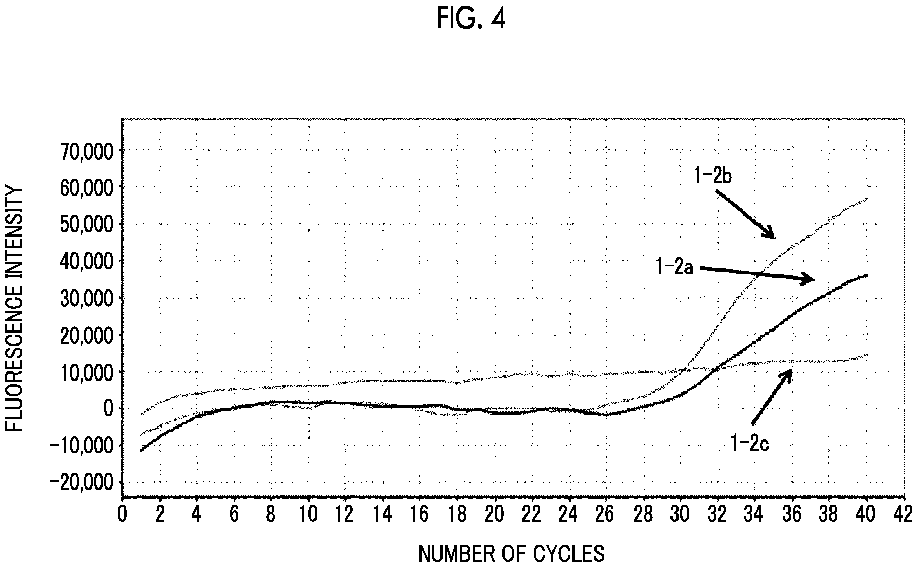

As is evident in the results of Figs. 4 (Comparative Example 1-2) to 9 (Comparative Example 1-7), in a case where a difference in the concentration (number of copies) was found between *Mycobacterium tuberculosis, Mycobacterium avium,* and *Mycobacterium intracellulare*, an increase in the fluorescence intensity derived from the bacterium present at the highest concentration (400 copies/µL) was confirmed, but an increase in the fluorescence intensity derived from the bacterium present at the lowest concentration (4 copies/µL) was not confirmed.

As shown in Figs. 4 (Comparative Examples 1-2) to 9 (Comparative Examples 1-7), it was confirmed that in a case where the method of the related art (method of Patent Literature 4) was used and a difference in the concentration (number of copies) was found between *Mycobacterium tuberculosis, Mycobacterium avium,* and *Mycobacterium intracellulare*, *Mycobacterium tuberculosis, Mycobacterium avium,* and *Mycobacterium intracellulare* were not able to be appropriately detected. In other words, it was found that the method of the related art (method of Patent Literature 4) is likely to cause false negatives.

### Example 3. Examination of concentration of each primer pair in primer set 3

The optimum concentration of each primer pair constituting the primer set 3 in Example 1 was examined.

### (1) Primer set

The primer set 3 used in Example 1 was used.

Here, in the primer pair for detecting *Mycobacterium tuberculosis,* the 5' terminal of the reverse primer consisting of the base sequence represented by SEQ ID NO: 2 was labeled with a labeling substance (TAMRA^{™}).

In the primer pair for detecting *Mycobacterium avium,* a linker (adenine (A)-adenine (A)) was added to the 5' terminal of the forward primer consisting of the base sequence represented by SEQ ID NO: 3, and the 5' terminal thereof was labeled with a labeling substance (TAMRA^{™}).

In addition, in the primer pair for detecting *Mycobacterium intracellulare*, a linker (adenine (A)-adenine (A)) was added to the 5' terminal of the forward primer consisting of the base sequence represented by SEQ ID NO: 5, and the 5' terminal thereof was labeled with a labeling substance (TAMRA^{™}).

Further, a primer pair (forward primer: base sequence represented by SEQ ID NO: 21, reverse primer: base sequence represented by SEQ ID NO: 22) for detecting an internal control (Bacillus subtilis) was used in combination with the primer set 3.

### (2) Preparation of template DNA

DNAs of *Mycobacterium tuberculosis, Mycobacterium avium,* and *Mycobacterium intracellulare* were respectively prepared in the same manner as in the section (2) of Example 1.

### (3) Nucleic acid amplification reaction/detection

A 10 mM Tris-HCl buffer solution (pH of 8.0) containing each primer pair at the concentration listed in Table 7, 1.5 mM of MgCl₂, 0.5 mg/mL of BSA, 0.2 mM of dATP, 0.2 mM of dCTP, 0.2 mM of dGTP, 0.2 mM of dTTP, and 60 U/mL of Takara Ex Taq (manufactured by Takara Bio Inc.) was prepared and used as a reaction solution for PCR.

It should be noted that the concentration ratios in Table 7 are the concentration ratios (%) of the primer pairs in the primer pair for detecting *Mycobacterium tuberculosis,* the primer pair for detecting *Mycobacterium avium,* and the primer pair for detecting *Mycobacterium intracellulare.*

**Table 7**

| | Concentration of primer pair for detecting Mycobacterium tuberculosis (concertration ratio) | Corcentration of primer pair for detecting Mycobacterium *avium* (concertration ratio) | Concentration of primer pair for detecting Mycobacterium intracellulare (concertration | Concentration of primer pair for detecting internal control |
|---|---|---|---|---|
| Example 3-1 | 400 nM (approximately 29%) | 600 nM (approximately 43%) | 400 nM (approximately 29%) | 220 nM |
| Example 3-2 | 400 nM (approximately 33%) | 400 nM (approximately 33%) | 400 nM (approximately 33%) | 220 nM |
| Example 3-3 | 200 nM (approximately 17%) | 600 nM (50%) | 400 nM (approximately 33%) | 220 nM |
| Example 3-4 | 400 nM (approximately 27%) | 600 nM (40%) | 500 nM (approximately 33%) | 220 nM |
| Example 3-5 | 400 nM (25%) | 600 nM (37.5%) | 600 nM (37.5%) | 220 nM |
| Example 3-6 | 400 nM (approximately 22%) | 800 nM (approximately 44%) | 600 nM (approximately 33%) | 220 nM |
| Example 3-7 | 300 nM (approximately 18%) | 800 nM (approximately 47%) | 600 nM (approximately 35%) | 220 nM |

Next, the DNA prepared in the section (2) was suspended in 25 µL of the reaction solution for PCR such that the concentration thereof reached 0.08 copy/µL and was used as a sample for PCR.

Thereafter, the nucleic acid amplification reaction (PCR) and electrophoresis were performed using the sample described above with a fully automated gene analysis device µTASWako g1 (manufactured by FUJIFILM Wako Pure Chemical Corporation) according to the instruction manual of the device.

### (4) Results

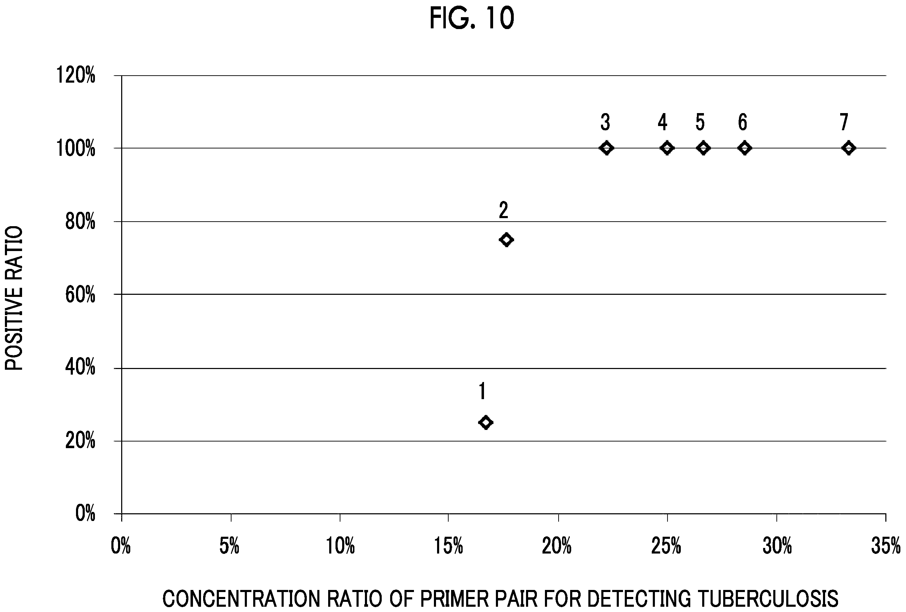

The results of the section (3) are respectively shown in Figs. 10 to 12. The vertical axis in Figs. 10 to 12 represents the positive ratio (%) in a case where the same example was measured a total of 8 times. In addition, the horizontal axis represents the concentration ratio of each primer pair in the primer set.

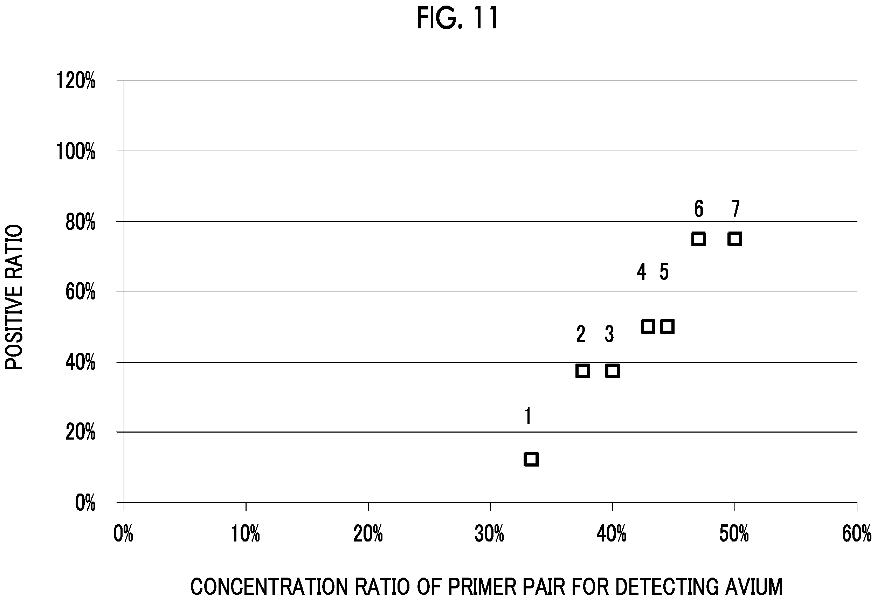

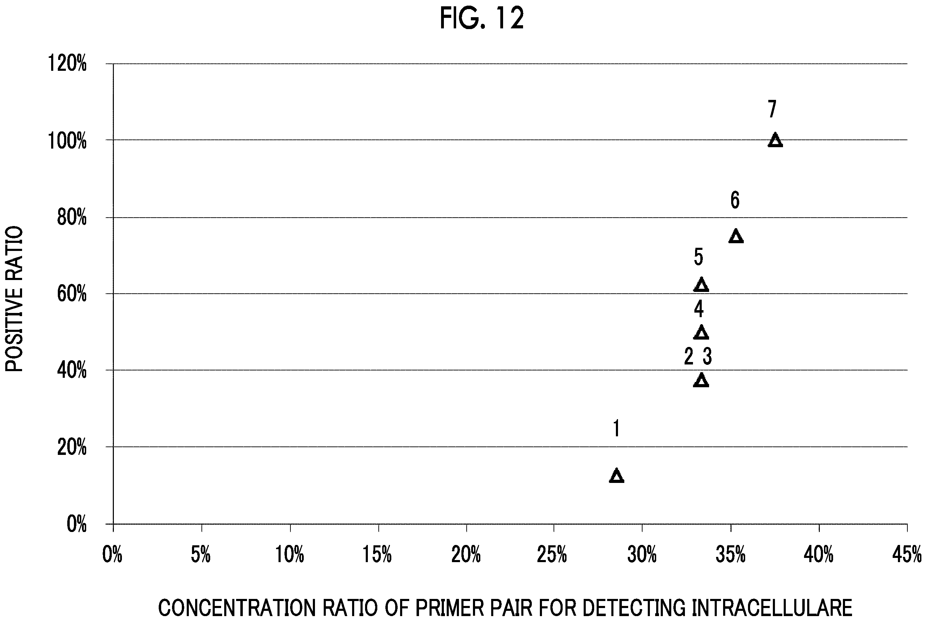

It should be noted that in each example, *Mycobacterium tuberculosis, Mycobacterium avium,* and *Mycobacterium intracellulare* were detected by the same reaction system, but the results are shown separately in Fig. 10 (*Mycobacterium tuberculosis*), Fig. 11 (*Mycobacterium avium*), and Fig. 12 (*Mycobacterium intracellulare*).

In addition, which of Examples 3-1 to 3-7 is represented by the number of each plot in Figs. 10 to 12 is as listed in Table 8.

**Table 8**

| | **Plot number in** **Fig. 10** | **Plot number in** **Fig. 11** | **Plot number in** **Fig. 12** |
|---|---|---|---|
| **Example 3-1** | 6 | 4 | 1 |
| **Example 3-2** | 7 | 1 | 2 |
| **Example 3-3** | 1 | 7 | 3 |
| **Example 3-4** | 5 | 3 | 4 |
| **Example 3-5** | 4 | 2 | 7 |
| **Example 3-6** | 3 | 5 | 5 |
| **Example 3-7** | 2 | 6 | 6 |

As is evident in Fig. 10 (Examples 3-1 to 3-7), Fig. 11 (Examples 3-1 to 3-7), and Fig. 12 (Examples 3-1 to 3-7), it was found that the positive ratio increases as the concentration ratio of each primer pair in the primer set increases.

Here, it was found that the clinically ideal conditions in which the positive ratios of *Mycobacterium tuberculosis, Mycobacterium avium,* and *Mycobacterium intracellulare* are the same as each other are the conditions of Example 3-7.

That is, the concentration ratio of the primer pair for detecting *Mycobacterium tuberculosis* is approximately 18% (300 nM) (2 in Fig. 10), the concentration ratio of the primer pair for detecting *Mycobacterium avium* is approximately 47% (800 nM) (6 in Fig. 11), and the concentration ratio of the primer pair for detecting *Mycobacterium intracellulare* is approximately 35% (600 nM) (6 in Fig. 12) as the conditions. Under such conditions, it was found that the positive ratios of *Mycobacterium tuberculosis, Mycobacterium avium,* and *Mycobacterium intracellulare* were respectively 75%, which was a satisfactory value.

Therefore, it was suggested that the primer set is suitable for use in actual clinical practice by setting the concentration ratio of the primer pair for detecting *Mycobacterium tuberculosis* to approximately 18% (300 nM), the concentration ratio of the primer pair for detecting *Mycobacterium avium* to approximately 47% (800 nM), and the concentration ratio of *Mycobacterium intracellulare* to approximately 35% (600 nM).

As shown in the results of Examples 1 to 3 and Comparative Example 1, it was found that the presence or absence of *Mycobacterium tuberculosis, Mycobacterium avium,* and *Mycobacterium intracellulare* can be specifically and accurately detected by using the primer set 3. In addition, it was found that the presence or absence of *Mycobacterium tuberculosis, Mycobacterium avium,* and *Mycobacterium intracellulare* can be specifically and accurately detected even in a case where a difference in the concentration (number of copies) is found between *Mycobacterium tuberculosis, Mycobacterium avium,* and *Mycobacterium intracellulare.*

### Industrial Applicability

According to the primer set for detecting *Mycobacterium tuberculosis, Mycobacterium avium,* and *Mycobacterium intracellulare* of the present invention, the method using the same, and the reagent kit therefor, it is possible to detect the genus Mycobacterium (*Mycobacterium tuberculosis, Mycobacterium avium,* and *Mycobacterium intracellulare*) present in a sample even in a case where a difference in concentration (number of copies) is found between the species of the genus Mycobacterium.

## Claims

1. A primer set comprising:
(i) a primer pair for detecting *Mycobacterium tuberculosis* which consists of a combination of a forward primer consisting of a base sequence represented by SEQ ID NO: 1 and a reverse primer consisting of a base sequence represented by SEQ ID NO: 2;
(ii) a primer pair for detecting *Mycobacterium avium* which consists of a combination of a forward primer consisting of a base sequence represented by SEQ ID NO: 3 and a reverse primer consisting of a base sequence represented by SEQ ID NO: 4; and
(iii) a primer pair for detecting *Mycobacterium intracellulare* which consists of a combination of a forward primer consisting of a base sequence represented by SEQ ID NO: 5 and a reverse primer consisting of a base sequence represented by SEQ ID NO: 6.

2. The primer set according to claim 1,
wherein at least one of the forward primers or the reverse primers of the primer pairs (i), (ii) and (iii) are respectively labeled with a labeling substance.

3. The primer set according to claim 2,
wherein the labeling substance is selected from a fluorescent substance, a radioactive isotope, or an enzyme.

4. A method of detecting *Mycobacterium tuberculosis, Mycobacterium avium,* and/or *Mycobacterium intracellulare*, comprising:
carrying out a nucleic acid amplification reaction using a nucleic acid in a sample as a template using the primer set according to claim 1; and
detecting an obtained nucleic acid amplification product.

5. A reagent kit comprising:
(i) a primer pair for detecting *Mycobacterium tuberculosis* which consists of a combination of a forward primer consisting of a base sequence represented by SEQ ID NO: 1 and a reverse primer consisting of a base sequence represented by SEQ ID NO: 2;
(ii) a primer pair for detecting *Mycobacterium avium* which consists of a combination of a forward primer consisting of a base sequence represented by SEQ ID NO: 3 and a reverse primer consisting of a base sequence represented by SEQ ID NO: 4; and
(iii) a primer pair for detecting *Mycobacterium intracellulare* which consists of a combination of a forward primer consisting of a base sequence represented by SEQ ID NO: 5 and a reverse primer consisting of a base sequence represented by SEQ ID NO: 6.

6. The reagent kit according to claim 5,
wherein at least one of the forward primers or the reverse primers of the primer pairs (i), (ii) and (iii) are respectively labeled with a labeling substance.


SEQUENCE LISTING

<110>  FUJIFILM Wako Pure Chemical Corporation

<120>  A primer set for detecting Mycobacterium tuberculosis,
       Mycobacterium avium and Mycobacterium intracellulare

<130>  2148PCT

<150>  2019-228461
<151>  2019-12-18

<160>  22

<170>  PatentIn version 3.5

<210>  1
<211>  20
<212>  DNA
<213>  Artificial Sequence

<220>
<223>  oligonucleotide primer

<400>  1
acctcaccta tgtgtcgacc                                                   20


<210>  2
<211>  22
<212>  DNA
<213>  Artificial Sequence

<220>
<223>  oligonucleotide primer

<400>  2
aacgtctttc aggtcgagta cg                                                22


<210>  3
<211>  19
<212>  DNA
<213>  Artificial Sequence

<220>
<223>  oligonucleotide primer

<400>  3
gtggaagggc aaaaacacc                                                    19


<210>  4
<211>  20
<212>  DNA
<213>  Artificial Sequence

<220>
<223>  oligonucleotide primer

<400>  4
tggaaacagg gaacaagacc                                                   20

<210>  5
<211>  19
<212>  DNA
<213>  Artificial Sequence

<220>
<223>  oligonucleotide primer

<400>  5
gcaggagatc catcaggaa                                                    19


<210>  6
<211>  18
<212>  DNA
<213>  Artificial Sequence

<220>
<223>  oligonucleotide primer

<400>  6
cacgccagac caagacga                                                     18


<210>  7
<211>  182
<212>  DNA
<213>  Mycobacterium tuberculosis

<400>  7
acctcaccta tgtgtcgacc tgggcagggt tcgcctacgt ggcctttgtc accgacgcct       60

acgctcgcag gatcctgggc tggcgggtcg cttccacgat ggccacctcc atggtcctcg      120

acgcgatcga gcaagccatc tggacccgcc aacaagaagg cgtactcgac ctgaaagacg      180

tt                                                                     182


<210>  8
<211>  152
<212>  DNA
<213>  Mycobacterium avium

<400>  8
tggaaacagg gaacaagacc gaaccgtatt ccgggctgga ctggcgatgc gcccagtggc       60

tgcatgacca cgaggtcgcc gcggtcgcct cggacaacct gcaggtcgaa gacccggtgt      120

ccggcgtcga cggggtgttt ttgcccttcc ac                                    152


<210>  9
<211>  213
<212>  DNA
<213>  Mycobacterium intracellulare

<400>  9
cacgccagac caagacgatc gacgaggtct tcaccggcaa gccgctgacc accatccccg       60

tcggcacggc cgaagacgtc gaagccgcct tcgccgaggc gcgcgcggcg caggccgact      120

gggcgaaccg tccggtcagc gagcgcgtcg atgtcatcag ccggtatcgc gacctggtcg 180

tcgagaaccg cgagttcctg atggatctcc tgc 213

<210> 10
<211> 21
<212> DNA
<213> Artificial Sequence

<220>
<223> oligonucleotide primer

<400> 10
tgggtagcag acctcaccta t 21

<210> 11
<211> 20
<212> DNA
<213> Artificial Sequence

<220>
<223> oligonucleotide primer

<400> 11
cgagtacgct ttcttgttgg 20

<210> 12
<211> 19
<212> DNA
<213> Artificial Sequence

<220>
<223> oligonucleotide primer

<400> 12
cgcacggatg tgattggta 19

<210> 13
<211> 20
<212> DNA
<213> Artificial Sequence

<220>
<223> oligonucleotide primer

<400> 13
cggagtagtg gacgaagagg 20

<210> 14
<211> 178
<212> DNA
<213> Mycobacterium tuberculosis

<400> 14
tgggtagcag acctcaccta tgtgtcgacc tgggcagggt tcgcctacgt ggcctttgtc 60

accgacgcct acgctcgcag gatcctgggc tggcgggtcg cttccacgat ggccacctcc 120

atggtcctcg acgcgatcga gcaagccatc tggacccgcc aacaagaagg cgtactcg 178

<210> 15
<211> 145
<212> DNA
<213> Mycobacterium avium

<400> 15
cgcacggatg tgattggtac atccgcattt gatgagagaa gtaagtaaat ggcacaggga 60

actgtgaaat ggttcaacgg tgaaaagggc ttcggcttca tcacccctga cgacggcacg 120

aaggacctct tcgtccacta ctccg 145

<210> 16
<211> 23
<212> DNA
<213> Artificial Sequence

<220>
<223> oligonucleotide primer

<400> 16
cacatgcaag tcgaacggaa agg 23

<210> 17
<211> 24
<212> DNA
<213> Artificial Sequence

<220>
<223> oligonucleotide primer

<400> 17
gcccgtatcg cccgcacgct caca 24

<210> 18
<211> 36
<212> DNA
<213> Artificial Sequence

<220>
<223> oligonucleotide probe

<400> 18
acgggatgca tgtcttgtgg tggaaagcgc tttagc 36

<210> 19
<211> 30
<212> DNA
<213> Artificial Sequence

<220>
<223> oligonucleotide probe

<400> 19
tttaggcgca tgtctttagg tggaaagctt 30

<210>  20
<211>  34
<212>  DNA
<213>  Artificial Sequence

<220>
<223>  oligonucleotide probe

<400>  20
tcaagacgca tgtcttctgg tggaaagctt ttgc                                    34


<210>  21
<211>  22
<212>  DNA
<213>  Artificial Sequence

<220>
<223>  oligonucleotide primer

<400>  21
cgcaatagag ggaagtggtt cc                                                 22


<210>  22
<211>  21
<212>  DNA
<213>  Artificial Sequence

<220>
<223>  oligonucleotide primer

<400>  22
cagcctccat aaggcttagc c                                                  21